# EUROPEAN PATENT APPLICATION

(11) **EP 4 582 084 A1**
(43) Date of publication of application: **09.07.2025**
(21) Application number: 23876507.7
(22) Date of filing: 22.09.2023
(51) Int. Cl.: A61K 31/4015, A61P 31/14

(54) **CRYSTAL FORM OF KETOAMIDE DERIVATIVE AND PREPARATION METHOD THEREFOR**

(30) Priority: 13.10.2022 CN 202211253246
(71) Applicant: Guangdong Raynovent Biotech Co., Ltd., Huangpu District Guangzhou 510700 (CN)
(72) Inventor: CHEN, Xiaoxin, Guangzhou, Guangdong 510700 (CN); LIU, Chengwu, Guangzhou, Guangdong 510700 (CN); LIU, Zhuowei, Guangzhou, Guangdong 510700 (CN); HUANG, Jianzhou, Guangzhou, Guangdong 510700 (CN); PANG, Daolin, Guangzhou, Guangdong 510700 (CN); LI, Bo, Guangzhou, Guangdong 510700 (CN); ZHOU, Guangqiang, Guangzhou, Guangdong 510700 (CN); CAI, Zemian, Guangzhou, Guangdong 510700 (CN); LONG, Chaofeng, Guangzhou, Guangdong 510700 (CN)
(74) Representative: Potter Clarkson
(86) International application number: PCT/CN2023/120726
(87) International publication number: WO 2024/078302

(57) **Abstract**

A crystal form of a ketoamide derivative and a preparation method therefor. The series of the crystal forms of the compound have good druggability (stability, fluidity, compressibility, solubility, bioavailability, and the like), and provide multiple active pharmaceutical ingredient choices for subsequent drug development.

## Description

### TECHNICAL FIELD

The present application belongs to the field of medicinal chemistry, and particularly relates to a series of crystal forms of a ketoamide derivative and a preparation method therefor, as well as an active pharmaceutical ingredient and a pharmaceutical composition comprising the crystal forms.

### BACKGROUND

SARS-CoV-2 (severe acute respiratory syndrome coronavirus-2), a highly pathogenic, pandemic zoonotic virus, belongs to the Coronaviridae family, along with SARS-CoV-1 and MERS-CoV. Unlike several other coronaviruses including HCoV-NL63, HCoV-229E, HCoV-OC43 and HCoVHKU1, these three viruses could cause severe respiratory diseases. The pathogen of COVID-19 is SARS-CoV-2, a member of coronaviridae, which can cause respiratory, hepatic, intestinal and neurological diseases in mammals. Humans infected with SARS-CoV-2 may present a variety of clinical manifestations, with the main common symptoms including fever, respiratory symptoms, cough, shortness of breath, etc., and the continued prolongation of infection may develop into severe pneumonia, causing serious complications such as respiratory failure, shock, and organ failure, and some of the patients with mild symptoms or discharged from the hospital may experience recurrence of the disease after recovery. What's more, this virus is not only highly contagious, but can be transmitted through asymptomatic infected individuals and those in the symptomatic and pre-symptomatic stages.

During coronavirus infection in a host, the enzymes essential for viral replication, the main proteases (M PRO, also known as 3CLPRO), are a class of cysteine hydrolases that are capable of cleaving polyproteins at multiple sites within the virus to generate multiple active functional proteins. The 3CLPRO sequence is highly conserved among coronaviruses and is crucial to the proper function of the coronaviruses. Inhibition of 3CLPRO can not only kill the coronaviruses effectively, but also reduce the immune imbalance within the infected host cells. Therefore, 3CLPRO is one of the key targets in the current development of broad-spectrum anti-coronaviral drugs, while 3CLPRO inhibitors have become an attractive object in the field of anti-viral chemotherapy.

The following compounds are the 3CLPRO inhibitor compounds that have been reported. S-217622 is an oral drug for treating novel coronavirus pneumonia developed by Shionogi & Co., Ltd., Japan, which can efficiently inhibit the novel coronavirus 3CLPRO and play an antiviral effect. It is demonstrated by preclinical trials that, S-217622 has a strong effect on the activity of 3CLPRO *in vitro,* with an IC50 value of 0.013 µM and an EC50 value of 0.37 µM. Paxlovid, an oral drug for novel coronavirus, is an oral drug for treating novel coronavirus pneumonia developed by Pfizer Inc., US, which is composed of 3CL protease inhibitors Nirmatrelvir (PF-07321332) and Ritonavir. This medicine, as the most effective oral drug currently on the market, has been validated in clinical trials to reduce the risk of death associated with novel coronavirus by 89%.

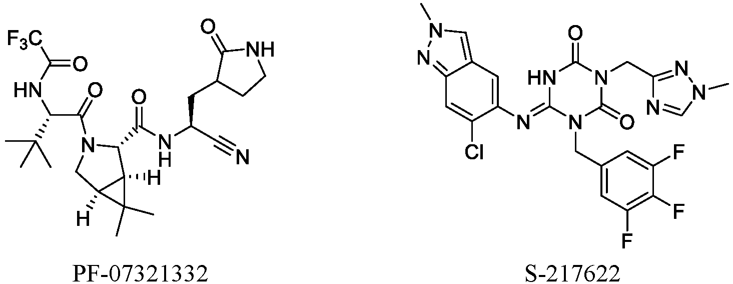

A series of ketoamide derivatives have been reported in Patent Application No. PCT/CN2022/117124. *In vitro* activity data show that some of the compounds have good *in vitro* anti-coronaviral activity at the cellular level without cytotoxicity and with significantly higher exposure, slower clearance rate, longer half-life and better pharmacokinetic properties. Among them, compound 1 (Example 1, Formula (I)) shows relatively outstanding overall performance and is considered to have better medicinal prospect.

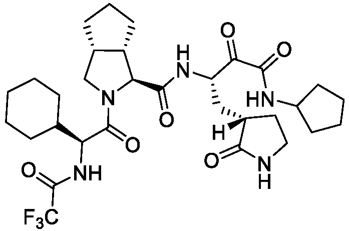

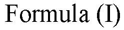

Crystal form screening is one of the most important aspects of drug development. Given a particular compound, the physicochemical properties of its free form, various salt forms, and corresponding crystal forms are not predictable. Based on further consideration on its druggability, it is of great significance to find suitable crystal forms to provide multiple intermediate and/or active pharmaceutical ingredient choices for subsequent drug development.

### SUMMARY OF THE INVENTION

The present application discloses a series of crystal forms of a compound of Formula (I). The series of crystal forms of the compound have good druggability (stability, fluidity, compressibility, solubility, bioavailability, and the like), and provide multiple active pharmaceutical ingredient choices for subsequent drug development.

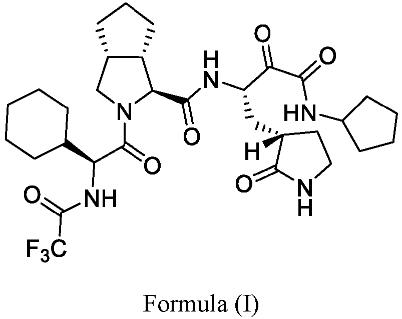

The above object of the present application is achieved through the following technical solution.

For the characterization of a crystal form of a compound, it is understood by those skilled in the art that, given a specific crystal form of a specific compound, due to the influence of instrumentation, operation methods, sample purity, human factors and other factors during the characterization process, there will be certain fluctuations in the 2θ angle of each diffraction peak in the X-ray powder diffraction (XRPD) pattern in the repeated experiments, and the range of fluctuations (error margin) is usually within a range of ±0.2°. In addition, it is also understood by those skilled in the art that, the stability and reproducibility of the diffraction peaks can be affected by a combination of the 2θ angle of each diffraction peak, the absorption intensity (peak height), and other factors of the X-ray powder diffraction pattern. Particularly, a diffraction peak with stronger absorption intensity, better separation and smaller 2θ angle has better stability and reproducibility, and the more applicable to be used to characterize the specific crystal form. However, for the diffraction peaks with larger 2θ angle and/or poorer separation and/or weaker relative intensity, they may fluctuate greatly due to the influence of instrumentation, operation methods, sample purity, human factors, etc., and may not be reproduced in repeated experiments, and therefore such absorption peaks are not diffraction peaks necessary for the characterization of a crystal form for those skilled in the art. More particularly, based on the common knowledge in the art on the characterization of a crystal form, the diffraction peaks in the present application are selected by comprehensively considering factors such as 2θ angle, absorption intensity (peak height), and the like, and are grouped according to the stability and reproducibility.

It is also understood by those skilled in the art that, the test results of the differential scanning calorimetric (DSC) curve and the thermal gravimetric analysis (TGA) curve of samples from the same batch and/or different batches may also fluctuate due to the influence of instrumentation, detection conditions, inspectors, etc. Therefore, based on the common knowledge in the art on the characterization of a crystal form, the fluctuation range of the starting points of the endothermic peak and the exothermic peak in the DSC pattern is set to ±3 °C, and the fluctuation range of the weight loss values in the TGA pattern is set to ±1%.

Unless otherwise specified, the "room temperature" in the present application means 25±5 °C, and "no significant weight loss shown" in the thermal gravimetric analysis (TGA) curve in the present application means a weight loss of ≤1% before the endpoint temperature of the detection.

It is understood by those skilled in the art that, at the completion of the preparation of the compound, the technician is not able to further investigate the specific form of the crystal form of the compound (I). Therefore, the compound of Formula (I) may exist in the form of anhydrate, hydrate, or solvate. The "solvent" in the solvate is an organic solvent which are commonly used in the art, including, but not limited to, methanol, ethanol, n-propanol, isopropanol, acetone, butanone, acetonitrile, dichloromethane, trichloromethane, ethyl acetate, toluene, etc.

A first object of the present application is to provide a crystal form I of a compound of Formula (I) and a preparation method therefor, and the crystal form shows good druggability.

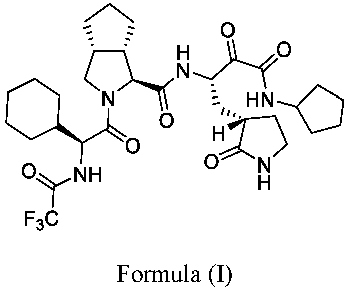

Particularly, there are diffraction peaks consistently observed at 2θ angles of 10.0, 10.6, 11.5, 12.1, 14.1, 16.7, 17.4, 19.0, 19.4, 20.5, 21.9, 24.9 (±0.2°) in an X-ray powder diffraction (XRPD) pattern of the crystal form I of the compound of Formula (I).

Further, there are also diffraction peaks at 2θ angles of 13.4, 14.5, 17.8, 18.7, 20.0, 21.1, 22.8, 23.8, 26.0, 26.9(±0.2°) in the XRPD pattern of the crystal form I of the compound of Formula (I).

Further, in some embodiments of the present application, the diffraction peaks in the XRPD pattern of the crystal form I of the compound of Formula (I) are as shown in the table below:

| No. | 2θ (±0.2°) | Peak height% | No. | 2θ (±0.2°) | Peak height% |
|---|---|---|---|---|---|
| 1 | 10.0 | 47.6 | 16 | 21.1 | 28.1 |
| 2 | 10.6 | 32.0 | 17 | 21.9 | 41.2 |
| 3 | 11.5 | 63.5 | 18 | 22.8 | 19.4 |
| 4 | 12.1 | 37.9 | 19 | 23.8 | 16.8 |
| 5 | 13.4 | 16.8 | 20 | 24.3 | 9.8 |
| 6 | 14.1 | 30.7 | 21 | 24.9 | 30.9 |
| 7 | 14.5 | 18.6 | 22 | 25.4 | 9.8 |
| 8 | 16.7 | 51.7 | 23 | 26.0 | 15.7 |
| 9 | 17.4 | 100.0 | 24 | 26.9 | 11.2 |
| 10 | 17.8 | 27.1 | 25 | 27.3 | 8.6 |
| 11 | 18.7 | 16.3 | 26 | 28.3 | 9.0 |
| 12 | 19.0 | 33.5 | 27 | 28.7 | 9.0 |
| 13 | 19.4 | 74.0 | 28 | 29.2 | 9.1 |
| 14 | 20.0 | 24.6 | 29 | 29.9 | 9.7 |
| 15 | 20.5 | 77.1 | | | |

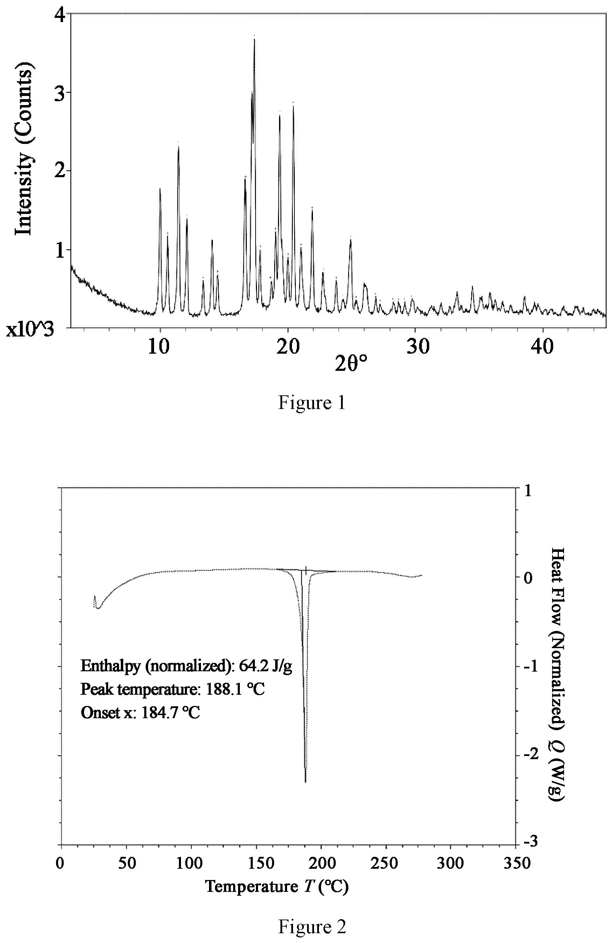

Further, in some embodiments of the present application, the XRPD pattern of the crystal form I of the compound of Formula (I) is essentially as shown in Figure 1.

There is a starting point of an endothermic peak at 188.1±3 °C on a differential scanning calorimetry (DSC) curve of the crystal form I of the compound of Formula (I).

Further, in some embodiments of the present application, the DSC pattern of the crystal form I of the compound of Formula (I) is essentially as shown in Figure 2.

There is a weight loss of 0.3±1% at 100 °C on a thermal gravimetric analysis (TGA) curve of the crystal form I of the compound of Formula (I).

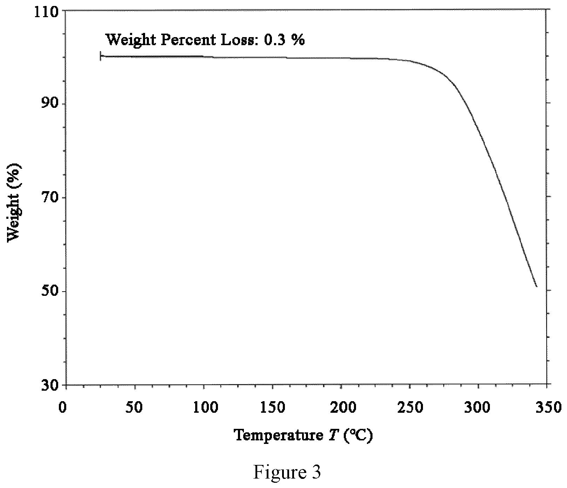

Further, in some embodiments of the present application, the TGA pattern of the crystal form I of the compound of Formula (I) is essentially as shown in Figure 3.

A second object of the present application is to provide a crystal form II of a compound of Formula (I) and a preparation method therefor, and the crystal form shows good druggability.

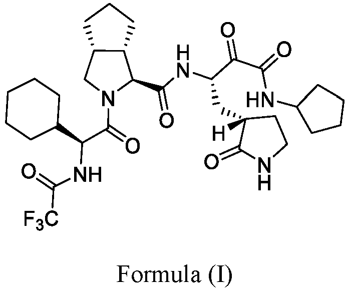

Particularly, there are diffraction peaks consistently observed at 2θ angles of 10.9, 12.1, 16.1, 17.0, 17.5, 18.3, 23.4 (±0.2°) in an X-ray powder diffraction (XRPD) pattern of the crystal form II of the compound of Formula (I).

Further, in some embodiments of the present application, the diffraction peak profile in the XRPD pattern of the crystal form II of the compound of Formula (I) is as shown in the table below:

| No. | 2θ (±0.2°) | Peak height% | No. | 2θ (±0.2°) | Peak height% |
|---|---|---|---|---|---|
| 1 | 10.9 | 48.2 | 5 | 17.5 | 70.5 |
| 2 | 12.1 | 40.3 | 6 | 18.3 | 100.0 |
| 3 | 16.1 | 55.3 | 7 | 23.4 | 51.9 |
| 4 | 17.0 | 68.4 | | | |

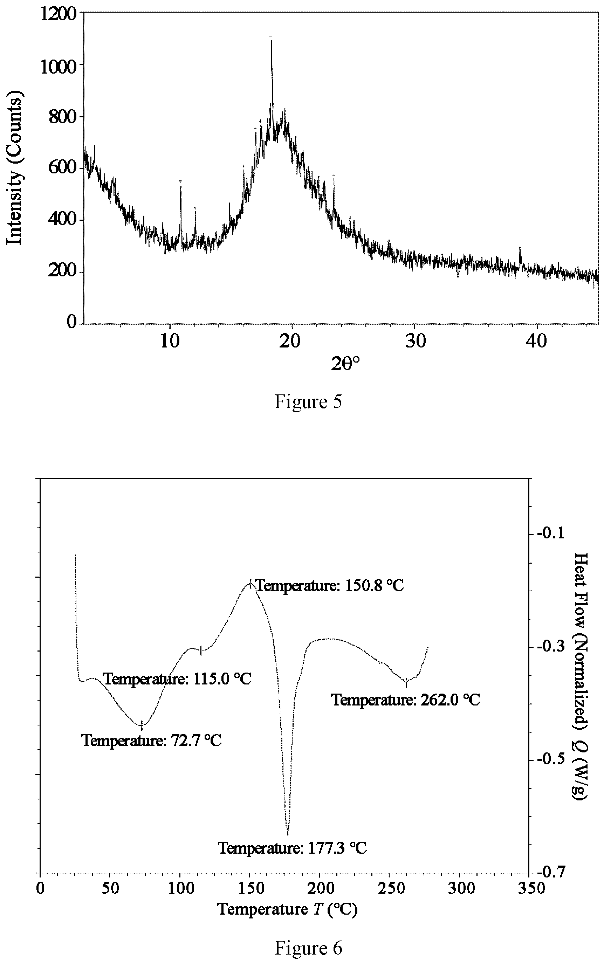

Further, in some embodiments of the present application, the XRPD pattern of the crystal form II of the compound of Formula (I) is essentially as shown in Figure 5.

There are starting points of endothermic peaks at 72.7, 115.0, 177.3, 262.0 (±3 °C) and a starting point of an exothermic peak at 150.8±3 °C on a differential scanning calorimetry (DSC) curve of the crystal form II of the compound of Formula (I).

Further, in some embodiments of the present application, the DSC pattern of the crystal form II of the compound of Formula (I) is essentially as shown in Figure 6.

There is a weight loss of 7.8±1% at 230 °C on a thermal gravimetric analysis (TGA) curve of the crystal form II of the compound of Formula (I).

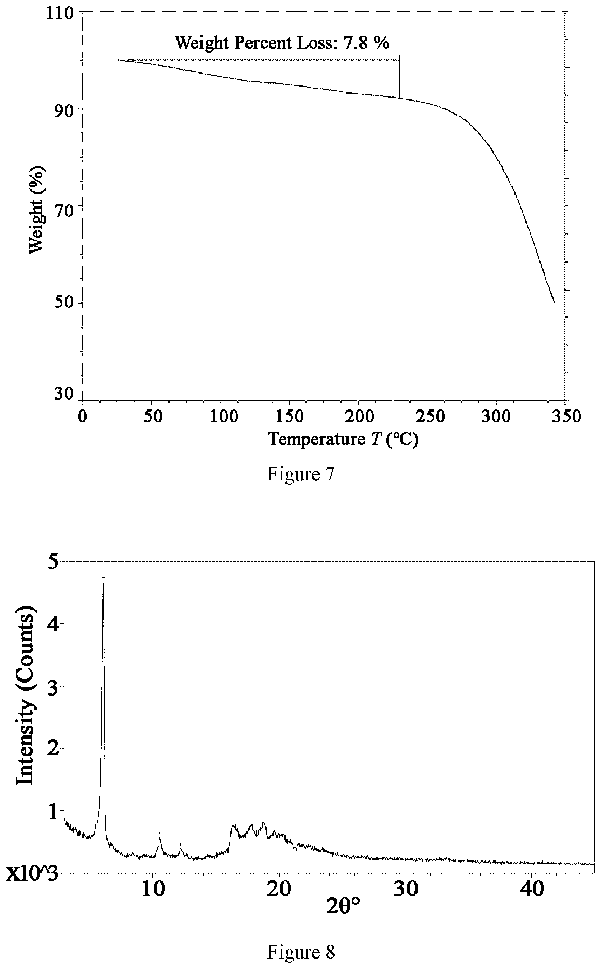

Further, in some embodiments of the present application, the TGA pattern of the crystal form II of the compound of Formula (I) is essentially as shown in Figure 7.

A third object of the present application is to provide a crystal form III of a compound of Formula (I) and a preparation method therefor, and the crystal form shows good druggability.

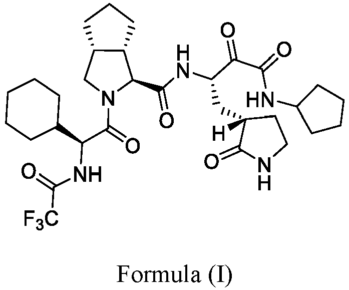

Particularly, there are diffraction peaks consistently observed at 2θ angles of 6.1, 10.6, 12.3, 16.6, 17.9, 18.7 (±0.2°) in an X-ray powder diffraction (XRPD) pattern of the crystal form III of the compound of Formula (I).

Further, in some embodiments of the present application, the diffraction peak profile in the XRPD pattern of the crystal form III of the compound of Formula (I) is as shown in the table below:

| No. | 2θ (±0.2°) | Peak height% | No. | 2θ (±0.2°) | Peak height% |
|---|---|---|---|---|---|
| 1 | 6.1 | 100.0 | 4 | 16.6 | 16.9 |
| 2 | 10.6 | 12.5 | 5 | 17.9 | 17.0 |
| 3 | 12.3 | 8.5 | 6 | 18.7 | 18.4 |

Further, in some embodiments of the present application, the XRPD pattern of the crystal form III of the compound of Formula (I) is essentially as shown in Figure 8.

There are starting points of endothermic peaks at 86.0, 130.8, 186.3 (±3 °C) on a differential scanning calorimetry (DSC) curve of the crystal form III of the compound of Formula (I).

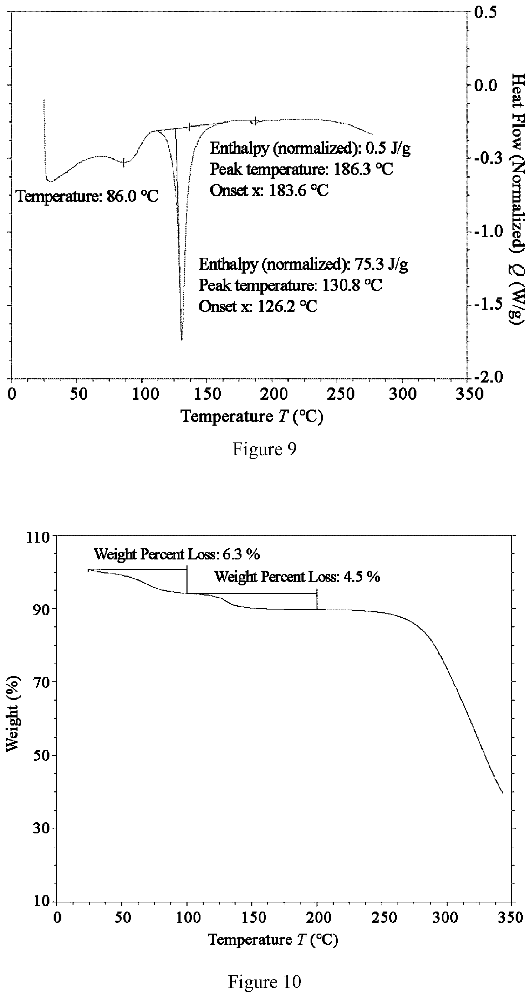

Further, in some embodiments of the present application, the DSC pattern of the crystal form III of the compound of Formula (I) is essentially as shown in Figure 9.

There is a weight loss of 6.3±1% at 100 °C and a weight loss of 4.5±1% at 200 °C on a thermal gravimetric analysis (TGA) curve of the crystal form III of the compound of Formula (I).

Further, in some embodiments of the present application, the TGA pattern of the crystal form III of the compound of Formula (I) is essentially as shown in Figure 10.

A fourth object of the present application is to provide a crystal form IV of a compound of Formula (I) and a preparation method therefor, and the crystal form shows good druggability.

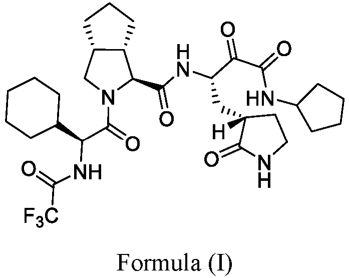

Particularly, there are diffraction peaks consistently observed at 2θ angles of 6.2, 8.1, 10.7, 16.4, 17.1, 18.6, 19.4, 21.3 (±0.2°) in an X-ray powder diffraction (XRPD) pattern of the crystal form IV of the compound of Formula (I).

Further, there are also diffraction peaks at 2θ angles of 7.1, 11.2, 16.7, 17.7, 18.8, 19.8, 20.4, 22.0, 22.3, 22.5, 23.1, 24.7 (±0.2°) in the XRPD pattern of the crystal form IV of the compound of Formula (I).

Further, in some embodiments of the present application, the diffraction peak profile in the XRPD pattern of the crystal form IV of the compound of Formula (I) is as shown in the table below:

| No. | 2θ (±0.2°) | Peak height% | No. | 2θ (±0.2°) | Peak height% |
|---|---|---|---|---|---|
| 1 | 6.2 | 100.0 | 14 | 18.6 | 25.6 |
| 2 | 7.1 | 9.3 | 15 | 18.8 | 14.1 |
| 3 | 8.1 | 13.3 | 16 | 19.4 | 10.8 |
| 4 | 10.7 | 22.4 | 17 | 19.8 | 9.8 |
| 5 | 11.2 | 7.2 | 18 | 20.4 | 8.9 |
| 6 | 11.9 | 4.9 | 19 | 21.3 | 10.6 |
| 7 | 12.8 | 5.6 | 20 | 22.0 | 8.0 |
| 8 | 13.4 | 5.5 | 21 | 22.3 | 8.7 |
| 9 | 14.1 | 4.4 | 22 | 22.5 | 9.6 |
| 10 | 16.4 | 33.3 | 23 | 23.1 | 8.7 |
| 11 | 16.7 | 13.2 | 24 | 24.7 | 6.2 |
| 12 | 17.1 | 15.0 | 25 | 27.0 | 5.2 |
| 13 | 17.7 | 7.9 | 26 | 28.4 | 4.8 |

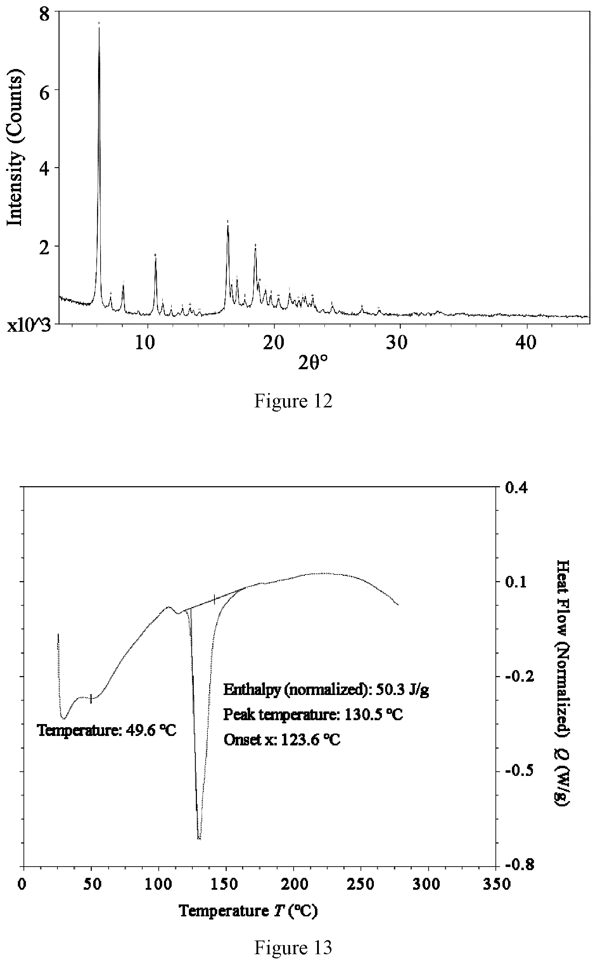

Further, in some embodiments of the present application, the XRPD pattern of the crystal form IV of the compound of Formula (I) is essentially as shown in Figure 12.

There are starting points of endothermic peaks at 49.6, 130.5±3 °C on a differential scanning calorimetry (DSC) curve of the crystal form IV of the compound of Formula (I).

Further, in some embodiments of the present application, the DSC pattern of the crystal form IV of the compound of Formula (I) is essentially as shown in Figure 13.

There is a weight loss of 3.8±1% at 110 °C and a weight loss of 4.2±1% at 190 °C on a thermal gravimetric analysis (TGA) curve of the crystal form IV of the compound of Formula (I).

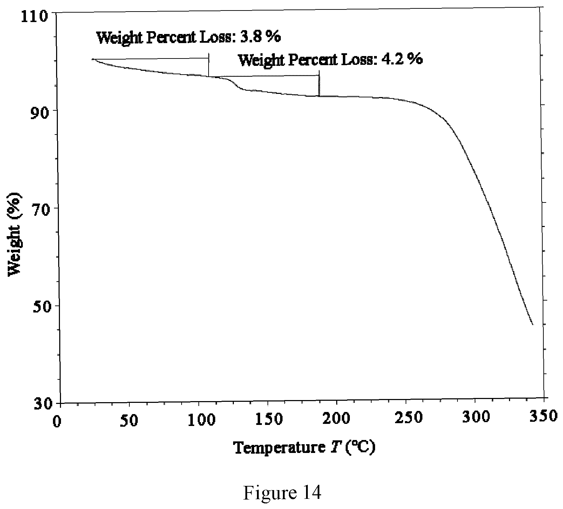

Further, in some embodiments of the present application, the TGA pattern of the crystal form IV of the compound of Formula (I) is essentially as shown in Figure 14.

A fifth object of the present application is to provide a crystal form V of a compound of Formula (I) and a preparation method therefor, and the crystal form shows good druggability.

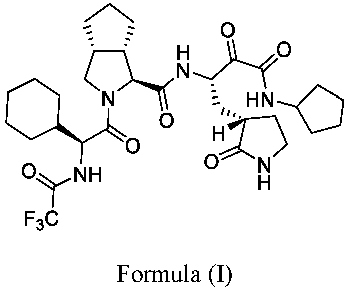

Particularly, there are diffraction peaks consistently observed at 2θ angles of 6.2, 8.1, 10.6, 16.2, 18.5, 19.1(±0.2°) in an X-ray powder diffraction (XRPD) pattern of the crystal form V of the compound of Formula (I).

Further, in some embodiments of the present application, the diffraction peak profile in the XRPD pattern of the crystal form V of the compound of Formula (I) is as shown in the table below:

| No. | 2θ (±0.2°) | Peak height% | No. | 2θ (±0.2°) | Peak height% |
|---|---|---|---|---|---|
| 1 | 6.2 | 100.0 | 4 | 16.2 | 16.9 |
| 2 | 8.1 | 9.3 | 5 | 18.5 | 12.1 |
| 3 | 10.6 | 14.3 | 6 | 19.1 | 12.1 |

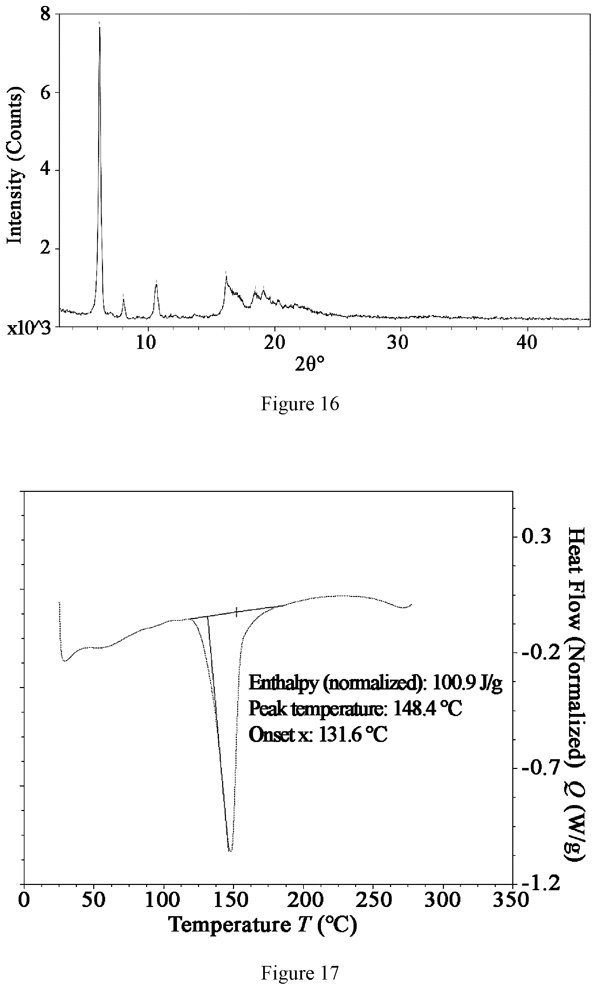

Further, in some embodiments of the present application, the XRPD pattern of the crystal form V of the compound of Formula (I) is essentially as shown in Figure 16.

There is a starting point of an endothermic peak at 148.4±3 °C on a differential scanning calorimetry (DSC) curve of the crystal form V of the compound of Formula (I).

Further, in some embodiments of the present application, the DSC pattern of the crystal form V of the compound of Formula (I) is essentially as shown in Figure 17.

There is a weight loss of 10.5±1% at 190 °C on a thermal gravimetric analysis (TGA) curve of the crystal form V of the compound of Formula (I).

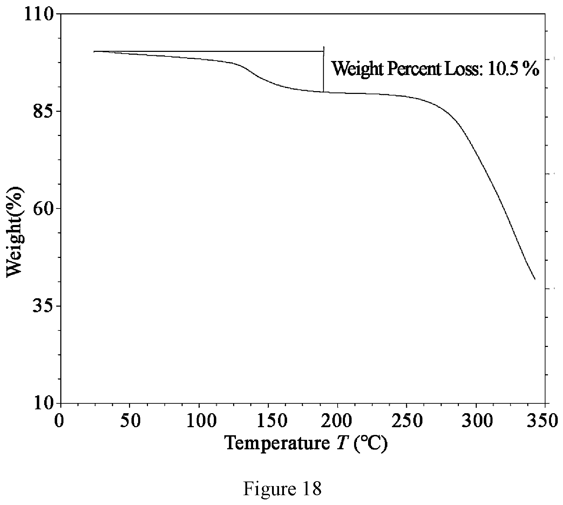

Further, in some embodiments of the present application, the TGA pattern of the crystal form V of the compound of Formula (I) is essentially as shown in Figure 18.

A sixth object of the present application is to provide a crystal form VI of a compound of Formula (I) and a preparation method therefor, and the crystal form shows good druggability.

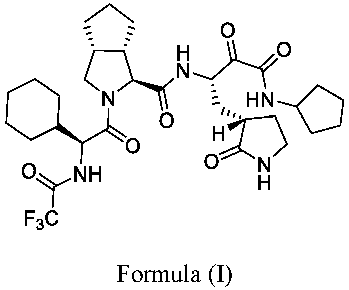

Particularly, there are diffraction peaks consistently observed at 2θ angles of 5.5, 5.8, 10.7, 16.9, 17.9, 18.3 (±0.2°) in an X-ray powder diffraction (XRPD) pattern of the crystal form VI of the compound of Formula (I).

Further, there are also diffraction peaks at 2θ angles of 9.1, 10.4, 15.9, 20.0, 20.9, 21.6 (±0.2°) in the XRPD pattern of the crystal form VI of the compound of Formula (I).

Further, in some embodiments of the present application, the diffraction peak profile in the XRPD pattern of the crystal form VI of the compound of Formula (I) is as shown in the table below:

| No. | 2θ (±0.2°) | Peak height% | No. | 2θ (±0.2°) | Peak height% |
|---|---|---|---|---|---|
| 1 | 5.5 | 100.0 | 7 | 16.9 | 27.4 |
| 2 | 5.8 | 59.2 | 8 | 17.9 | 27.8 |
| 3 | 9.1 | 15.8 | 9 | 18.3 | 24.9 |
| 4 | 10.4 | 19.4 | 10 | 20.0 | 18.0 |
| 5 | 10.7 | 20.3 | 11 | 20.9 | 17.6 |
| 6 | 15.9 | 17.1 | 12 | 21.6 | 16.9 |

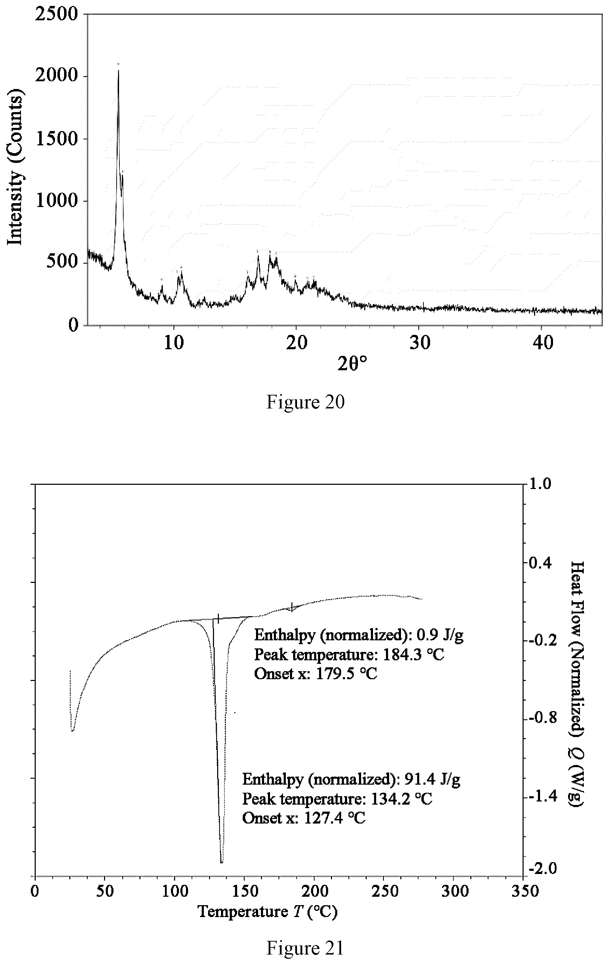

Further, in some embodiments of the present application, the XRPD pattern of the crystal form VI of the compound of Formula (I) is essentially as shown in Figure 20.

There are starting points of endothermic peaks at 134.2, 184.3 (±3 °C) on a differential scanning calorimetry (DSC) curve of the crystal form VI of the compound of Formula (I).

Further, in some embodiments of the present application, the DSC pattern of the crystal form VI of the compound of Formula (I) is essentially as shown in Figure 21.

There is a weight loss of 16.2±1% at 200 °C on a thermal gravimetric analysis (TGA) curve of the crystal form VI of the compound of Formula (I).

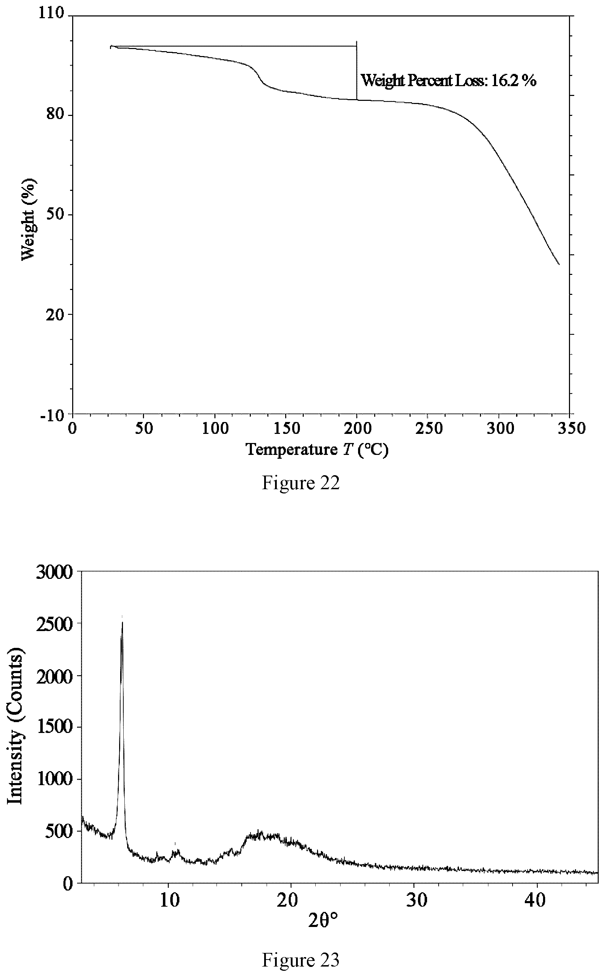

Further, in some embodiments of the present application, the TGA pattern of the crystal form VI of the compound of Formula (I) is essentially as shown in Figure 22.

A seventh object of the present application is to provide a crystal form VII of a compound of Formula (I) and a preparation method therefor, and the crystal form shows good druggability.

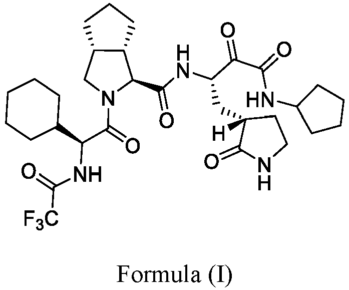

Particularly, there are diffraction peaks consistently observed at 2θ angles of 6.3, 10.9 (±0.2°) in an X-ray powder diffraction (XRPD) pattern of the crystal form VII of the compound of Formula (I).

Further, in some embodiments of the present application, the XRPD pattern of the crystal form VII of the compound of Formula (I) is essentially as shown in Figure 23.

There is a starting point of an endothermic peak at 130.2±3 °C on a differential scanning calorimetry (DSC) curve of the crystal form VII of the compound of Formula (I).

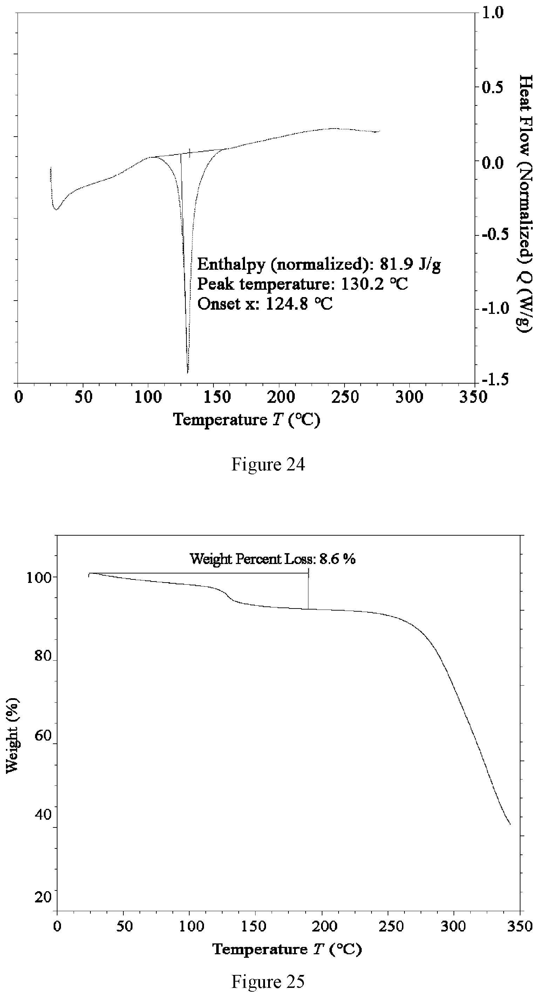

Further, in some embodiments of the present application, the DSC pattern of the crystal form VII of the compound of Formula (I) is essentially as shown in Figure 24.

There is a weight loss of 8.6±1% at 190 °C on a thermal gravimetric analysis (TGA) curve of the crystal form VII of the compound of Formula (I).

Further, in some embodiments of the present application, the TGA pattern of the crystal form VII of the compound of Formula (I) is essentially as shown in Figure 25.

An eighth object of the present application is to provide a crystal form VIII of a compound of Formula (I) and a preparation method therefor.

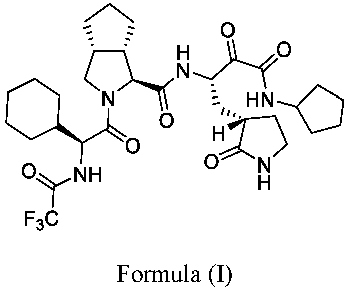

Particularly, there are diffraction peaks consistently observed at 2θ angles of 5.3, 11.7, 15.8, 16.6, 17.4, 18.8, 20.3 (±0.2°) in an X-ray powder diffraction (XRPD) pattern of the crystal form VIII of the compound of Formula (I).

Further, in some embodiments of the present application, the diffraction peak profile in the XRPD pattern of the crystal form VIII of the compound of Formula (I) is as shown in the table below:

| No. | 2θ (±0.2°) | Peak height% | No. | 2θ (±0.2°) | Peak height% |
|---|---|---|---|---|---|
| 1 | 5.3 | 100.0 | 5 | 17.4 | 13.9 |
| 2 | 11.7 | 5.6 | 6 | 18.8 | 17.7 |
| 3 | 15.8 | 8.8 | 7 | 20.3 | 12.2 |
| 4 | 16.6 | 16.2 | | | |

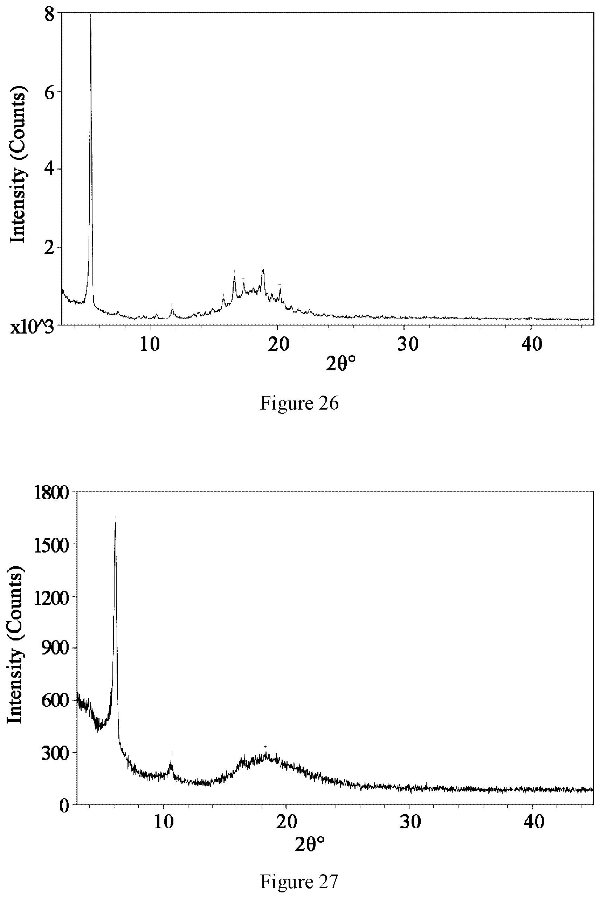

Further, in some embodiments of the present application, the XRPD pattern of the crystal form VIII of the compound of Formula (I) is essentially as shown in Figure 26.

A ninth object of the present application is to provide a crystal form IX of a compound of Formula (I) and a preparation method therefor.

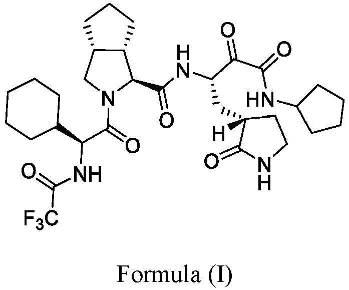

Particularly, there are diffraction peaks consistently observed at 2θ angles of 6.1, 10.6, 16.6 (±0.2°) in an X-ray powder diffraction (XRPD) pattern of the crystal form IX of the compound of Formula (I).

Further, in some embodiments of the present application, the diffraction peak profile in the XRPD pattern of the crystal form IX of the compound of Formula (I) is as shown in the table below:

| No. | 2θ (±0.2°) | Peak height% |
|---|---|---|
| 1 | 6.1 | 100.0 |
| 2 | 10.6 | 15.7 |
| 3 | 16.6 | 18.0 |

Further, in some embodiments of the present application, the XRPD pattern of the crystal form IX of the compound of Formula (I) is essentially as shown in Figure 27.

A tenth object of the present application is to provide an active pharmaceutical ingredient comprising at least one of the crystal forms I to IX of the compound of Formula (I) of the present application.

Based on the beneficial effects of the crystal forms I to IX of the compound of Formula (I) of the present application, the active pharmaceutical ingredients containing the crystal forms also exhibit substantially consistent beneficial effects (e.g., stability, water solubility, etc.) with the crystal forms. Particularly, the active pharmaceutical ingredients may be the compound of Formula (I), or the compound of Formula (I) and/or a hydrate of the compound of Formula (I), or the compound of Formula (I) and/or an anhydrate of the compound of Formula (I), or the compound of Formula (I) and/or a solvate of the compound of Formula (I). More particularly, a mass percentage of the crystal form I of the compound of Formula (I) and/or the crystal form II of the compound of Formula (I) and/or the crystal form III of the compound of Formula (I) and/or the crystal form IV of the compound of Formula (I) and/or the crystal form V of the compound of Formula (I) and/or the crystal form VI of the compound of Formula (I) and/or the crystal form VII of the compound of Formula (I) and/or the crystal form VIII of the compound of Formula (I) and/or the crystal form IX of the compound of Formula (I) contained in the active pharmaceutical ingredient is any value from 0.01% to 99.99%. Further, the mass percentage of the crystal form I of the compound of Formula (I) and/or the crystal form II of the compound of Formula (I) and/or the crystal form III of the compound of Formula (I) and/or the crystal form IV of the compound of Formula (I) and/or the crystal form V of the compound of Formula (I) and/or the crystal form VI of the compound of Formula (I) and/or the crystal form VII of the compound of Formula (I) and/or the crystal form VIII of the compound of Formula (I) and/or the crystal form IX of the compound of Formula (I) contained in the active pharmaceutical ingredient is any value from 1.00% to 99.00%.

An eleventh object of the present application is to provide a pharmaceutical composition composed of the active pharmaceutical ingredient and a pharmaceutically acceptable excipient. The pharmaceutically acceptable excipient comprises but not limited to, at least one of filler, binder, disintegrant, and lubricant. Particularly, based on the beneficial effects of the crystal forms I to IX of the compound of Formula (I) of the present application, the beneficial effects are ultimately reflected in the pharmaceutical composition. More particularly, the pharmaceutical composition comprises a mass percentage of any value from 1.00% to 99.00% of the active pharmaceutical ingredient. Further, the pharmaceutical composition comprises a mass percentage of any value from 5.00% to 95.00% of the active pharmaceutical ingredient. Furthermore, the pharmaceutical composition comprises a mass percentage of any value from 10.00% to 90.00% of the active pharmaceutical ingredient.

A twelfth object of the present application is to provide a drug comprising at least one of the crystal forms or the active pharmaceutical ingredient or the pharmaceutical composition.

A thirteenth object of the present application is to provide use of a pharmaceutical composition in the preparation of a drug for treating coronavirus infection. Particularly, the coronavirus is HCoV-229E, HCoV-OC43, HCoV-NL63, HCoV-HKU1, SARS-CoV, MERS-CoV, SARS-CoV-2, or a variant thereof.

In summary, the crystal forms I to IX of the compound of Formula (I) of the present application have certain medicinal prospect. Therefore, if it is demonstrated by detection means that the crystal forms I to IX of the compound of Formula (I) of the present application exist in the active pharmaceutical ingredient and/or in the pharmaceutical composition, it shall be deemed that the crystal forms I to IX of the compound of Formula (I) as provided in the present application have been used. In addition to X-ray powder diffraction as mentioned above, the detection means may further include such methods as Differential Scanning Calorimetry (DSC), Infrared Spectroscopy (IR), Raman Spectrometry (Raman), Solid State Nuclear Magnetic Resonance (SSNMR), as well as all other detection methods that, individually or in combination, can be used to demonstrate the use of the crystal forms I to IX of the compound of Formula (I) of the present application, and the effects resulted from pharmaceutical excipients can be removed by using methods commonly used by those skilled in the art, such as subtractive pattern.

With respect to the prior art, the present application has the following advantages and beneficial effects:
1. Firstly disclosing a crystal form I of a compound of Formula (I) and a preparation method therefor, and this crystal form has the properties including high stability and has a considerable medicinal prospect.
2. Firstly disclosing a crystal form II of a compound of Formula (I) and a preparation method therefor, and this crystal form has the properties including high stability and has a considerable medicinal prospect.
3. Firstly disclosing a crystal form III of a compound of Formula (I) and a preparation method therefor, and this crystal form has the properties including high stability and has a considerable medicinal prospect.
4. Firstly disclosing a crystal form IV of a compound of Formula (I) and a preparation method therefor, and this crystal form has the properties including high stability and has a considerable medicinal prospect.
5. Firstly disclosing a crystal form V of a compound of Formula (I) and a preparation method therefor, and this crystal form has the properties including high stability and has a considerable medicinal prospect.
6. Firstly disclosing a crystal form VI of a compound of Formula (I) and a preparation method therefor, thus providing multiple intermediate and/or active pharmaceutical ingredient choices for the scale production of active pharmaceutical ingredients and the downstream process (e.g., formulation process) of pharmaceutical products.
7. Firstly disclosing a crystal form VII of a compound of Formula (I) and a preparation method therefor, thus providing multiple intermediate and/or active pharmaceutical ingredient choices for the scale production of active pharmaceutical ingredients and the downstream process (e.g., formulation process) of pharmaceutical products.
8. Firstly disclosing a crystal form VIII of a compound of Formula (I) and a preparation method therefor, thus providing multiple intermediate and/or active pharmaceutical ingredient choices for the scale production of active pharmaceutical ingredients and the downstream process (e.g., formulation process) of pharmaceutical products.
9. Firstly disclosing a crystal form IX of a compound of Formula (I) and a preparation method therefor, thus providing multiple intermediate and/or active pharmaceutical ingredient choices for the scale production of active pharmaceutical ingredients and the downstream process (e.g., formulation process) of pharmaceutical products.
10. Providing an active pharmaceutical ingredient comprising at least one of crystal forms I to IX of the compound of Formula (I) of the present application, which has substantially consistent beneficial effects with the crystal forms I to IX of the compound of Formula (I) of the present application.
11. Providing a pharmaceutical composition composed of an active pharmaceutical ingredient of the present application and a pharmaceutically acceptable excipient, which has substantially consistent beneficial effects with the crystal forms I to IX of the compound of Formula (I) of the present application.

### BRIEF DESCRIPTION OF THE DRAWINGS

Figure 1 shows an XRPD pattern of the crystal form I of the compound of Formula (I).
Figure 2 shows a DSC pattern of the crystal form I of the compound of Formula (I).
Figure 3 shows a TGA pattern of the crystal form I of the compound of Formula (I).
Figure 4 shows an XRPD comparison diagram of the crystal form I of the compound of Formula (I).
Figure 5 shows an XRPD pattern of the crystal form II of the compound of Formula (I).
Figure 6 shows a DSC pattern of the crystal form II of the compound of Formula (I).
Figure 7 shows a TGA pattern of the crystal form II of the compound of Formula (I).
Figure 8 shows an XRPD pattern of the crystal form III of the compound of Formula (I).
Figure 9 shows a DSC pattern of the crystal form III of the compound of Formula (I).
Figure 10 shows a TGA pattern of the crystal form III of the compound of Formula (I).
Figure 11 shows an XRPD comparison diagram of the crystal form III of the compound of Formula (I).
Figure 12 shows an XRPD pattern of the crystal form IV of the compound of Formula (I).
Figure 13 shows a DSC pattern of the crystal form IV of the compound of Formula (I).
Figure 14 shows a TGA pattern of the crystal form IV of the compound of Formula (I).
Figure 15 shows an XRPD comparison diagram of the crystal form IV of the compound of Formula (I).
Figure 16 shows an XRPD pattern of the crystal form V of the compound of Formula (I).
Figure 17 shows a DSC pattern of the crystal form V of the compound of Formula (I).
Figure 18 shows a TGA pattern of the crystal form V of the compound of Formula (I).
Figure 19 shows an XRPD comparison diagram of the crystal form V of the compound of Formula (I).
Figure 20 shows an XRPD pattern of the crystal form VI of the compound of Formula (I).
Figure 21 shows a DSC pattern of the crystal form VI of the compound of Formula (I).
Figure 22 shows a TGA pattern of the crystal form VI of the compound of Formula (I).
Figure 23 shows an XRPD pattern of the crystal form VII of the compound of Formula (I).
Figure 24 shows a DSC pattern of the crystal form VII of the compound of Formula (I).
Figure 25 shows a TGA pattern of the crystal form VII of the compound of Formula (I).
Figure 26 shows an XRPD pattern of the crystal form VIII of the compound of Formula (I).
Figure 27 shows an XRPD pattern of the crystal form IX of the compound of Formula (I).

### DETAILED DESCRIPTION

The present application will be further described in detail below with reference to the embodiments and attached drawings, but the embodiments of the present application are not limited thereto.

### Detection conditions

X-ray powder diffraction (XRPD)
   X-ray powder diffractometer: Bruker D8 Advance;
   Scanning 2θ angle range: from 3° to 45°;
   Scanning step size: 0.02°;
   Exposure time: 0.2 sec;
   Tube voltage and current: 40 KV, 40 mA.
Differential Scanning Calorimetry (DSC)
   Differential scanning calorimeter: TA Discovery 2500 (TA, US);
   Heating rate: 10 °C/min;
   Detection method: The sample is precisely weighed and placed in a DSC Tzero sample plate, heated to 350 °C while purging with nitrogen in the furnace at a rate of 50 mL/min.
Thermogravimetric Analysis (TGA)
   Thermogravimetric analyzer: TA Discovery 55(TA, US);
   Detection method: The sample is placed in a balanced open aluminum sample plate and weighed automatically in a heating furnace; and the sample is heated to 400 °C at a rate of 10 °C/min, and purged with nitrogen at a rate of 60 mL/min at the sample location and 40 mL/min at the balance location.

### Example 1: Preparation method of compound of Formula (I)

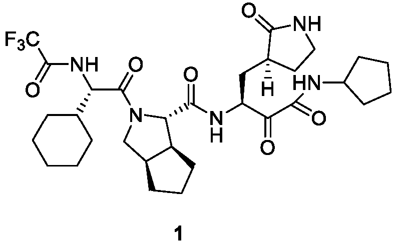

### Synthetic route:

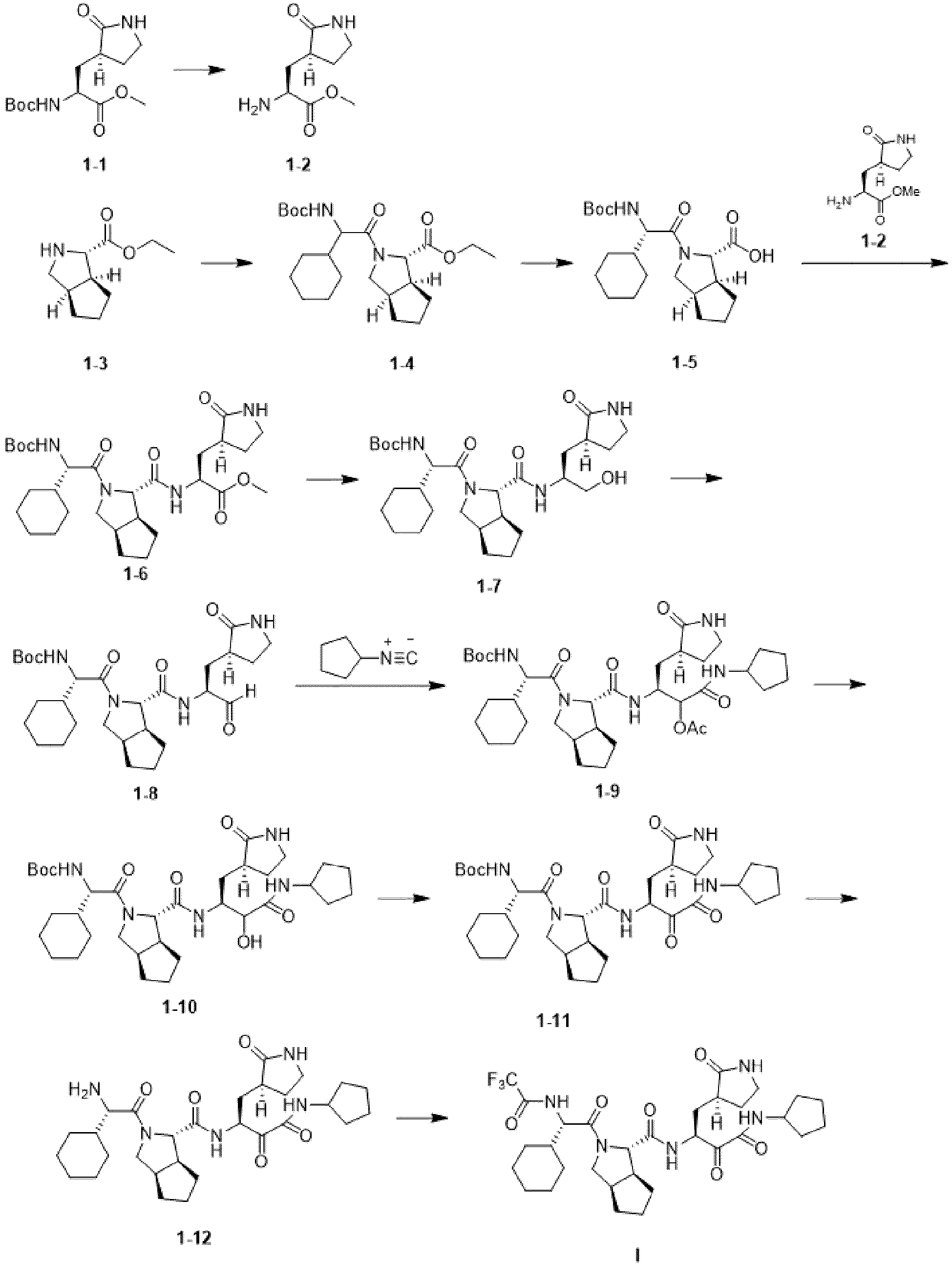

### Step 1: Synthesis of hydrochloride of Compound 1-2

Compound 1-1 (500 mg, 1.75 mmol) was dissolved in ethyl acetate (5 mL), into which was added a solution of hydrogen chloride in ethyl acetate (10 mL, 4 N) to react while stirring at 20°C for 2 h. The reaction solution was concentrated under reduced pressure to obtain a hydrochloride of Compound 1-2, without purification. ¹H NMR (400 MHz, CD₃OD) δ = 4.28 - 4.20 (m, 1H), 3.91 - 3.81 (m, 3H), 3.45 - 3.35 (m, 2H), 2.86 - 2.74 (m, 1H), 2.48 - 2.36 (m, 1H), 2.29 - 2.19 (m, 1H), 2.02 - 1.94 (m, 1H), 1.93 - 1.80 (m, 1H).

### Step 2: Synthesis of Compound 1-4

A compound Boc-L-cyclohexyl glycine (1 g, 3.89 mmol) was added into N,N-dimethylformamide (10 mL), into which was added 2-(7-azabenzotriazol-1-yl)-*N,N,N,N-*tetramethyluronium hexafluorophosphate (1.77 g, 4.66 mmol) to react while stirring for 0.5 h, and then added diisopropylethylamine (1.26 g, 9.72 mmol) and the hydrochloride of Compound 1-3 (1.02 g, 4.66 mmol) to react while stirring at 20 °C for 16 h. Into the reaction solution was added methyl tert-butyl ether (50 mL), and then the reaction solution was washed with water (20 mL), 3% citric acid (20 mL ×2), and saturated sodium chloride solution (20 mL). The organic layer was dried over anhydrous sodium sulfate, filtered, concentrated under reduced pressure, and purified by silica gel column chromatography (petroleum ether: ethyl acetate = 3:1) to obtain Compound 1-4. ¹H NMR (400MHz, CDCl₃) δ = 5.22 - 5.11 (m, 1H), 4.36 (d, J=3.9 Hz, 1H), 4.27 (dd, J=6.9, 9.3 Hz, 1H), 4.21 - 4.12 (m, 2H), 3.83 (dd, J=7.8, 10.4 Hz, 1H), 3.70 (br dd, J=3.6, 10.4 Hz, 1H), 2.81 - 2.61 (m, 2H), 1.82 - 1.70 (m, 6H), 1.68 - 1.61 (m, 4H), 1.56 - 1.48 (m, 2H), 1.46 - 1.38 (m, 9H), 1.29 - 1.22 (m, 4H), 1.21 - 0.98 (m, 4H).

### Step 3: Synthesis of Compound 1-5

Compound 1-4 (1.41 g, 3.34 mmol) was added into tetrahydrofuran (14 mL), into which was added a solution of lithium hydroxide monohydrate LiOH·H₂O (280.03 mg, 6.67 mmol) in water (5 mL) to react while stirring at 20 °C for 16 h. The crude product was neutralized with 3% citric acid solution (50 mL), and extracted with ethyl acetate (50 mL). The organic phase was washed with saturated sodium chloride solution (30 mL). The organic layer was dried over anhydrous sodium sulfate, filtered, and concentrated under reduced pressure to obtain Compound 1-5, without purification. ¹H NMR (400MHz, DMSO-d₆) δ = 12.58 - 12.23 (m, 1H), 6.92 - 6.82 (m, 1H), 4.11 - 3.94 (m, 2H), 3.82 - 3.76 (m, 1H), 3.72 - 3.62 (m, 1H), 2.73 - 2.64 (m, 1H), 2.62 - 2.55 (m, 1H), 1.92 - 1.42 (m, 12H), 1.40 - 1.32 (m, 9H), 1.18 - 1.06 (m, 3H), 1.00 - 0.81 (m, 2H).

### Step 4: Synthesis of Compound 1-6

Compound 1-5 (650 mg, 1.65 mmol) was added into 2-butanone (7 mL), into which were added 1-hydroxybenzotriazole (222.63 mg, 1.65 mmol), 1-(3-dimethylaminopropyl)-3-ethylcarbodiimidehydrochloride (379.03 mg, 1.98 mmol), and diisopropylethylamine (638.84 mg, 4.94 mmol) to react while stirring at 20 °C for 0.5 h, and then added the hydrochloride of Compound 1-2 (366.88 mg, 1.65 mmol) to react while stirring at 20°C for 16 h. Into the reaction solution was added water (20 mL), and then the reaction solution was extracted with dichloromethane: methanol (30 mL×2, 10:1). The organic phase was combined, and washed with 3% citric acid (20 mL×2) and saturated sodium chloride solution (20 mL). The organic layer was dried over anhydrous sodium sulfate, filtered, concentrated under reduced pressure, and purified by silica gel column chromatography (dichloromethane: methanol= 20: 1) to obtain Compound 1-6. ¹H NMR (400 MHz, CDCl₃) δ = 7.49 - 7.42 (m, 1H), 6.23 - 6.05 (m, 1H), 5.28 - 5.17 (m, 1H), 4.64 - 4.51 (m, 1H), 4.43 - 4.24 (m, 2H), 3.92 - 3.81 (m, 1H), 3.78 - 3.70 (m, 3H), 3.39 - 3.27 (m, 2H), 2.94 - 2.75 (m, 2H), 2.57 - 2.36 (m, 2H), 2.24 - 2.07 (m, 1H), 1.94 - 1.50 (m, 14H), 1.49 - 1.41 (m, 9H), 1.27 - 0.95 (m, 6H).

### Step 5: Synthesis of Compound 1-7

Compound 1-6 (3.10 g, 5.51 mmol) was dissolved in tetrahydrofuran (31 mL), into which was added lithium borohydride (240.02mg, 11.02mmol) at 0 °C and warmed to 20 °C slowly to react for 2 h. Into the reaction solution were added water (10 mL) and ethyl acetate (20 mL), and the reaction solution was stirred for 10 min. White solid was precipitated and filtered to obtain a filter cake, which was a crude product of Compound 1-7. [M+1]⁺ = 535.4.

### Step 6: Synthesis of Compound 1-8

Compound 1-7 (0.5 g, 935.13 µmol) was dissolved in dichloromethane (10 mL), and Dess-Martin periodinane (594.94mg, 1.40mmol) was then added into the reaction system to react while stirring at 25 °C for 16 h. Saturated sodium thiosulfate (15 mL) and saturated sodium bicarbonate solution (15 mL) were added into the reaction system, which was then stirred for 10 min and extracted with dichloromethane (50 mL ×2). The organic phase was washed with saturated salt water (15 mL), dried over anhydrous sodium sulfate, filtered, and concentrated to obtain a crude product of Compound 1-8. [M+1]⁺ = 533.4.

### Step 7: Synthesis of Compound 1-9

Compound 1-8 (436 mg, 818.52 µmol) was dissolved in dichloromethane (5 mL). Glacial acetic acid (58.98 mg, 982.22 mmol) and cyclopentyl isocyanate (94.44 mg, 982.22 µmol) were added into the reaction system to react while stirring at 25 °C for 2h. Saturated ammonium chloride solution (10 mL) was added into the reaction system, which was then stirred for 10 min and extracted with dichloromethane (20 mL). The organic phase was washed with water (10 mL), dried over anhydrous sodium sulfate, filtered, concentrated under reduced pressure, and purified by silica gel column chromatography (dichloromethane: methanol =10: 1) to obtain Compound 1-9. [M+1]⁺ = 688.4.

### Step 8: Synthesis of Compound 1-10

Compound 1-9 (190 mg, 276.22 µmol) was dissolved in methanol (3 mL), into which was then added a solution of potassium carbonate (95.44 mg, 690.54 µmol) in water (2 mL) to react while stirring at 20 °C for 16 h. 3% citric acid (20 mL) was added into the reaction system, which was then extracted with dichloromethane (40 mL) three times. The organic phase was washed with saturated sodium chloride solution (30 mL), dried over anhydrous sodium sulfate, filtered, and concentrated under reduced pressure to obtain a crude product of Compound 1-10. [M+1]⁺ = 646.5.

### Step 9: Synthesis of Compound 1-11

Compound 1-10 (238.00 mg, 368.52 µmol) was dissolved in dichloromethane (24 mL), into which was then added Dess-Martin periodinane (203.19 mg, 479.08 µmol) to react while stirring at 20 °C for 18 h. Sodium thiosulfate (15 mL) and sodium bicarbonate solution (15 mL) were added into the reaction system, which was then stirred for 10 min and extracted with dichloromethane (50 mL ×2). The organic phase was washed with saturated salt water (15 mL), dried over anhydrous sodium sulfate, filtered, concentrated, and purified by silica gel column chromatography (dichloromethane: methanol= 20:1) to obtain the product of Compound 1-11. [M+1]⁺ = 644.5.

### Step 10: Synthesis of Compound 1-12

Compound 1-11 (125 mg, 194.16 µmol) was dissolved in tetrahydrofuran (3 mL), into which was then added ethyl acetate hydrochloride (4 mol/L, 2.91 mL) to react while stirring at 20 °C for 1 h. The reaction solution was directly rotary evaporated with an oil pump, and then rotary evaporated again with a small amount of dichloromethane to obtain Compound 1-12. [M+1]⁺ =544.4.

### Step 11: Synthesis of Compound of Formula (I)

Compound 1-12 (125 mg, 229.91 µmol) was dissolved in tetrahydrofuran (2.5 mL) at 0 °C, into which was then added trifluoroacetic anhydride (193.15 mg, 919.63 µmol) and pyridine (127.30 mg, 1.61 mmol) to react while stirring at 20 °C for 16 h. Into the reaction system was added water (20 mL), and the reaction system was extracted with dichloromethane (40 mL ×2). The organic phase was washed with 3% citric acid (40 mL) and saturated sodium chloride solution (40 mL ×2) successively, dried over anhydrous sodium sulfate, filtered, and concentrated. The crude product was separated by preparative HPLC to obtain Compound of Formula (I). [M+1]⁺ = 640.0, ¹H NMR (400 MHz, CD₃OD) δ ppm 0.94 - 1.10 (m, 2 H), 1.13 - 1.32 (m, 3 H) 1.32 - 1.46 (m, 1 H), 1.47 - 1.57 (m, 3 H), 1.59 - 1.68 (m, 4 H), 1.69 - 1.81 (m, 6 H), 1.83 - 2.00 (m, 5 H), 2.01 - 2.17 (m, 1 H), 2.19 - 2.38 (m, 1 H), 2.49 - 2.57 (m, 1 H), 2.58 - 2.70 (m, 1 H), 2.73 - 2.89 (m, 1 H), 3.20 - 3.26 (m, 1 H), 3.37 - 3.45 (m, 1 H), 3.73 - 3.86 (m, 1 H), 3.88 - 3.97 (m, 1 H), 4.03 - 4.10 (m, 1 H), 4.11 - 4.18 (m, 1 H), 4.19 - 4.29 (m, 1 H), 4.29 - 4.37 (m, 1 H), 4.39 - 4.47 (m, 1 H), 4.57 - 4.60 (m, 2 H).

### Example 2: Preparation method of crystal form I of the compound of Formula (I)

20.0 mg of the compound of Formula (I) prepared by using the method of Example 1 was weighed and added into 0.5 mL of acetone/n-heptane (v/v, 1:9) to prepare a suspension, which was suspended while stirring at room temperature (about 25 °C) for 7 days. The suspension was centrifuged, and dried at room temperature under vacuum to obtain a white solid, i.e., crystal form I, of which the XRPD pattern was as shown in Figure 1, the DSC pattern was as shown in Figure 2, and the TGA pattern was as shown in Figure 3.

### Example 3: Preparation method of crystal form I of the compound of Formula (I)

50.2 mg of the compound of Formula (I) prepared by using the method of Example 1 was weighed and added into 0.5 mL of acetone/isopropyl ether (v/v, 1:4) to prepare a suspension, which was suspended while stirring at 10 °C for 24 h. The suspension was centrifuged, and dried at room temperature under vacuum to obtain a white solid, i.e., crystal form I.

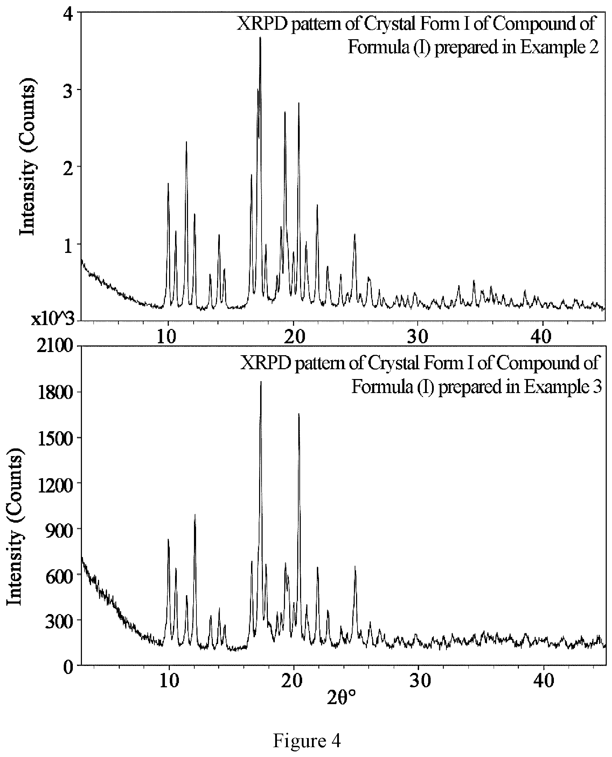

The XRPD comparison diagram of the resulting crystal form I was as shown in Figure 4.

### Example 4: Preparation method of crystal form II of the compound of Formula (I)

40.1 mg of the compound of Formula (I) prepared by using the method of Example 1 was weighed and mixed with 2.0 mL of cyclohexane at 50°C to form a suspension, into which was gradually dropwise added 4.2 mL of preheated toluene until the solid was just completely dissolved or mostly dissolved, and the suspension was filtered while hot, after then the resulting solution was transferred to cool at room temperature. After leaving to stand at room temperature for over 2 h, no sufficient solid precipitated. The solution was placed at 4 °C for further cooling, no sufficient solid precipitated, and the solution was placed at -15 °C for further cooling. The system was centrifuged to precipitate sufficient solid, and the solid was dried at room temperature under vacuum to get a white solid, i.e., crystal form II, of which the XRPD pattern was as shown in Figure 5, the DSC pattern was as shown in Figure 6, and the TGA pattern was as shown in Figure 7.

### Example 5: Preparation method of crystal form III of the compound of Formula (I)

19.9 mg of the compound of Formula (I) prepared by using the method of Example 1 was weighed and added into 0.5 mL of methanol/isopropyl ether (v/v, 1:4) to get a suspension, which was suspended while stirring at 10 °C for 24 h. The suspension was centrifuged, and the resulting solution was dried at room temperature under vacuum to get a white solid, i.e., crystal form III, of which the XRPD pattern was as shown in Figure 8, the DSC pattern was as shown in Figure 9, and the TGA pattern was as shown in Figure 10.

### Example 6: Preparation method of crystal form III of the compound of Formula (I)

20.0 mg of the compound of Formula (I) prepared by using the method of Example 1 was weighed and mixed with 1.0 mL of isopropyl ether at 50 °C to form a suspension, into which was gradually dropwise added 0.1 mL of preheated methanol until the solid was just completely dissolved or mostly dissolved, and the suspension was filtered while hot, after then the resulting solution was transferred to cool at room temperature. After leaving to stand at room temperature for over 2 h, the system was centrifuged to precipitate sufficient solid, and the solid was dried at room temperature under vacuum to get a white solid, i.e., crystal form III.

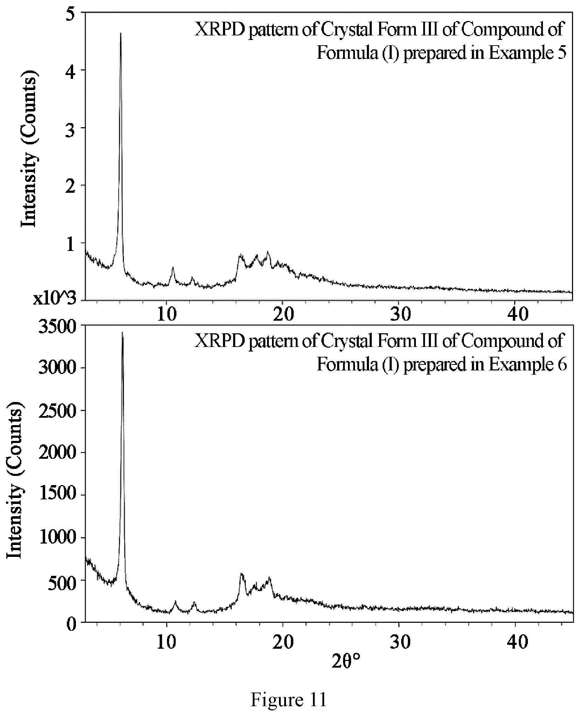

The XRPD comparison diagram of the resulting crystal form III was as shown in Figure 11.

### Example 7: Preparation method of crystal form IV of the compound of Formula (I)

19.5 mg of the compound of Formula (I) prepared by using the method of Example 1 was dissolved in 0.1 mL of ethanol to get a clear solution, which was left open at room temperature until the solvent volatilized completely to get a solid. The solid was dried at room temperature under vacuum to get a white solid, i.e., crystal form IV, of which the XRPD pattern was as shown in Figure 12, the DSC pattern was as shown in Figure 13, and the TGA pattern was as shown in Figure 14.

### Example 8: Preparation method of crystal form IV of the compound of Formula (I)

20.6 mg of the compound of Formula (I) prepared by using the method of Example 1 was weighed and added into 0.5 mL of ethanol/cyclohexane (v/v, 1:9) to get a suspension, which was suspended while stirring at room temperature (about 25 °C) for 7 days. The suspension was centrifuged, and the resulting solution was dried at room temperature under vacuum to get a white solid, i.e., crystal form IV.

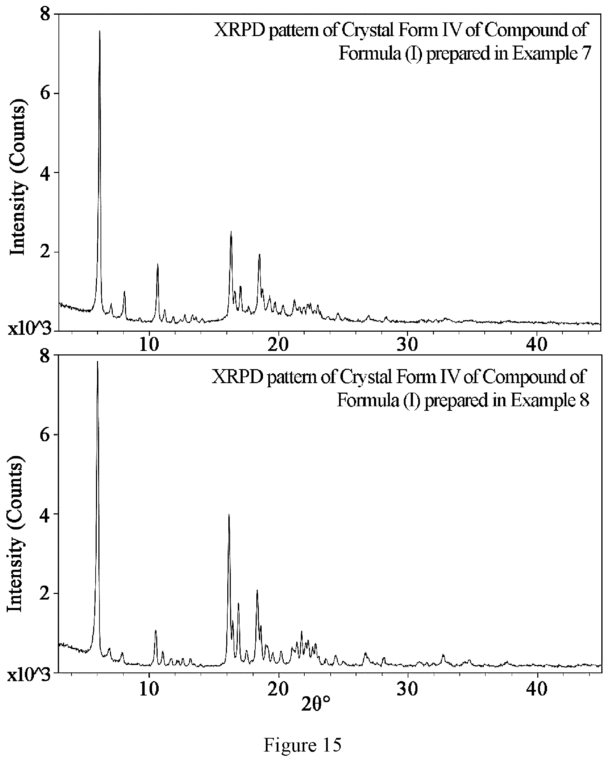

The XRPD comparison diagram of the resulting crystal form IV was as shown in Figure 15.

### Example 9: Preparation method of crystal form V of the compound of Formula (I)

19.5 mg of the compound of Formula (I) prepared by using the method of Example 1 was dissolved in 0.1 mL of n-propanol to get a clear solution, which was left open at room temperature until the solvent volatilized completely to get a solid. The solid was dried at room temperature under vacuum to get a white solid, i.e., crystal form V, of which the XRPD pattern was as shown in Figure 16, the DSC pattern was as shown in Figure 17, and the TGA pattern was as shown in Figure 18.

### Example 10: Preparation method of crystal form V of the compound of Formula (I)

19.4 mg of the compound of Formula (I) prepared by using the method of Example 1 was weighed and added into 0.5 mL of n-propanol/water (v/v, 1:9) to get a suspension, which was suspended while stirring at room temperature (about 25 °C) for 7 days. The suspension was centrifuged, and the resulting solution was dried at room temperature under vacuum to get a white solid, i.e., crystal form V.

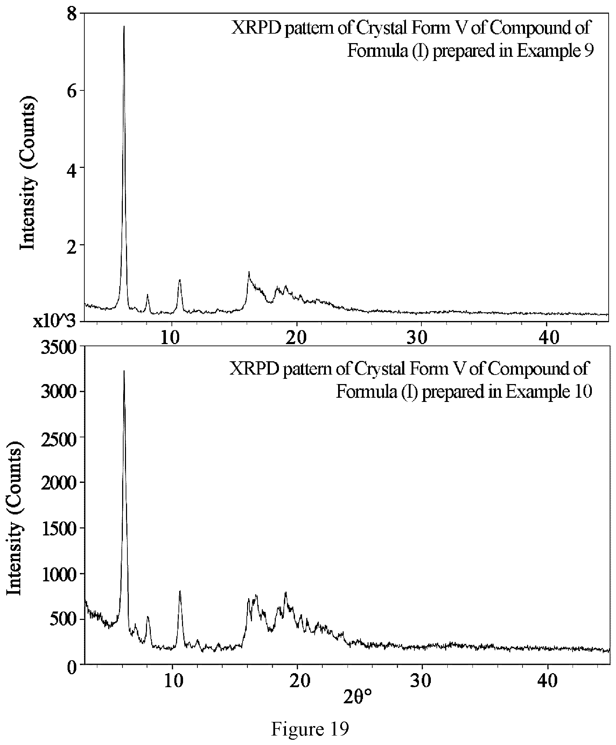

The XRPD comparison diagram of the resulting crystal form V was as shown in Figure 19.

### Example 11: Preparation method of crystal form VI of the compound of Formula (I)

20.2 mg of the compound of Formula (I) prepared by using the method of Example 1 was weighed, into which was dropwise added a certain amount (0.1 mL) of 2-methoxyethanol at room temperature to absolutely dissolve the sample, and then dropwise added 1.0 mL of isopropyl ether until a solid precipitated. After stirring at room temperature for 1 h, the system was centrifuged to precipitate a solid, and the solid was dried at room temperature under vacuum to get a white solid, i.e., crystal form VI, of which the XRPD pattern was as shown in Figure 20, the DSC pattern was as shown in Figure 21, and the TGA pattern was as shown in Figure 22.

### Example 12: Preparation method of crystal form VII of the compound of Formula (I)

20.0 mg of the compound of Formula (I) prepared by using the method of Example 1 was weighed and mixed with 1.0 mL of cyclohexane at 50 °C to form a suspension, into which was gradually dropwise added 0.4 mL of preheated chloroform until the solid was just completely dissolved or mostly dissolved, and the suspension was filtered while hot, after then the resulting solution was transferred to cool at room temperature. After leaving to stand at room temperature for over 2 h, no sufficient solid precipitated. The solution was placed at 4 °C for further cooling, no sufficient solid precipitated, and the solution was placed at -15 °C for further cooling. The system was centrifuged to precipitate sufficient solid, and the solid was dried at room temperature under vacuum to get a white solid, i.e., crystal form VII, of which the XRPD pattern was as shown in Figure 23, the DSC pattern was as shown in Figure 24, and the TGA pattern was as shown in Figure 25.

### Example 13: Preparation method of crystal form VIII of the compound of Formula (I)

20.5 mg of the compound of Formula (I) prepared by using the method of Example 1 was weighed and mixed with 1.0 mL of cyclohexane at 50 °C to form a suspension, into which was gradually dropwise added 0.05 mL of preheated n-propanol until the solid was just completely dissolved or mostly dissolved, and the suspension was filtered while hot, after then the resulting solution was transferred to cool at room temperature. After leaving to stand at room temperature for over 2 h, there was a solid precipitated. The system was centrifuged to precipitate sufficient solid, and the solid was dried at room temperature under vacuum to get a white solid, i.e., crystal form VIII, of which the XRPD pattern was as shown in Figure 26. Upon comparison, the crystal form VIII was essentially consistent with the crystal form of the compound of Formula (I) prepared by using the method of Example 1. The resulting crystal form VIII has a certain degree of hygroscopicity, and the powder is poor in shape and difficult to shape.

### Example 14: Preparation method of crystal form IX of the compound of Formula (I)

19.8 mg of the compound of Formula (I) prepared by using the method of Example 1 was weighed and mixed with 1.0 mL of n-heptane at 50 °C to form a suspension, into which was gradually dropwise added 0.9 mL of preheated butyl formate until the solid was just completely dissolved or mostly dissolved, and the suspension was filtered while hot, after then the resulting solution was transferred to cool at room temperature. After leaving to stand at room temperature for over 2 h, no sufficient solid precipitated. The solution was placed at 4°C for further cooling, no sufficient solid precipitated, and the solution was placed at -15°C for further cooling. The system was centrifuged to precipitate sufficient solid, and the solid was dried at room temperature under vacuum to get a white solid, i.e., crystal form IX, of which the XRPD pattern was as shown in Figure 27.

### Example 15: Test on the solid stability of crystal form I, crystal form IV and crystal form V of the compound of Formula (I) under conditions of high temperature and high humidity

2 samples of the crystal forms I, IV and V of the compound of Formula (I) were weighed in parallel, approximately 100 mg each, and placed in the bottom of a glass sample vial and spread into a thin layer. The sample vial was sealed with aluminum foil having small holes poked therein to ensure that the samples can be in full contact with the ambient air, and placed in a box with constant temperature and humidity at conditions of 40 °C/ 75% humidity. The samples placed under the above conditions were tested on Days 0, 12, and 30, and the test results were compared with the initial test result on Day 0 and shown in Table 1 to Table 3 below.

**Table 1. Test on the solid stability of the crystal form I of the compound of Formula (I)**

| Holding conditions | | | Day 0 | High temperature (60°C) | | High temperature/high humidity (40 °C/75% RH) | | High humidity (92.5% RH) | |
|---|---|---|---|---|---|---|---|---|---|
| Holding time | | | | Day 12 | Day 30 | Day 12 | Day 30 | Day 12 | Day 30 |
| Characteristics | Appearance | Should be white to yellowish powder | White powder | White powder | White powder | White powder | White powder | White powder | White powder |
| Detection | Total impurities | ≤5.0% | 0.36 | 0.66 | 0.83 | 0.69 | 0.72 | 0.60 | 0.81 |
| | Water | ≤5.0% | 0.55 | 0.56 | 0.55 | 0.66 | 0.69 | 0.90 | 0.83 |
| Crystal form | | Should be consistent with Day 0 | - | Consistent with Day 0 | Consistent with Day 0 | Consistent with Day 0 | Consistent with Day 0 | Consistent with Day 0 | Consistent with Day 0 |
| Weight gain after moisture absorption | | Reported value % | N/A | N/A | N/A | N/A | N/A | 0.72 | 0.56 |

| | | | | | | | | | |
|---|---|---|---|---|---|---|---|---|---|
| * N/A means not detected | | | | | | | | | |

**Table 2. Test on the solid stability of the crystal form IV of the compound of Formula (I)**

| Holding conditions | | | Day 0 | High temperature (60 °C) | | High temperature/high humidity (40 °C/75% RH) | | High humidity (92.5% RH) | |
|---|---|---|---|---|---|---|---|---|---|
| Holding time | | | | Day 12 | Day 30 | Day 12 | Day 30 | Day 12 | Day 30 |
| Characteristics | Appearance | Should be white to yellowish powder | White powder | White powder | White powder | White powder | White powder | White powder | White powder |
| Detection | Total impurities | ≤5.0% | 0.65 | 0.51 | 0.62 | 0.58 | 0.63 | 0.81 | 0.92 |
| | Water | ≤5.0% | 1.84 | 1.80 | 1.76 | 3.92 | 3.75 | 4.92 | 7.19 |
| Crystal form | | Should be consistent with Day 0 | - | Consistent with Day 0 | Consistent with Day 0 | Consistent with Day 0 | Consistent with Day 0 | Consistent with Day 0 | Consistent with Day 0 |
| Weight gain after moisture absorption | | Reported value % | N/A | N/A | N/A | N/A | N/A | 1.9 | 2.0 |

| | | | | | | | | | |
|---|---|---|---|---|---|---|---|---|---|
| * N/A means not detected | | | | | | | | | |

**Table 3. Test on the solid stability of the crystal form V of the compound of Formula (I)**

| Holding conditions | | | Day 0 | High temperature (60 °C) | | High temperature/high humidity (40 °C/75% RH) | | High humidity (92.5% RH) | |
|---|---|---|---|---|---|---|---|---|---|
| Holding time | | | | 12 days | 30 days | 12 days | 30 days | 12 days | 30 days |
| Characteristics | Appearance | Should be white to yellowish powder | White powder | White powder | White powder | White powder | White powder | White powder | White powder |
| Detection | Total impurities | ≤5.0% | 0.95 | 1.4 | 1.4 | 1.7 | 1.5 | 2.1 | 1.7 |
| | Water | ≤5.0% | 1.15 | 0.78 | 0.77 | 3.71 | 3.44 | 2.43 | 4.15 |
| Crystal form | | Should be consistent with Day 0 | - | Consistent with Day 0 | Consistent with Day 0 | Consistent with Day 0 | Consistent with Day 0 | Consistent with Day 0 | Consistent with Day 0 |
| Weight gain after moisture absorption | | Reported value % | N/A | N/A | N/A | N/A | N/A | 1.0 | 1.1 |

| | | | | | | | | | |
|---|---|---|---|---|---|---|---|---|---|
| * N/A means not detected | | | | | | | | | |

The detection items of the sample were compared with those on Day 0.

After holding the crystal form I of the compound of Formula I at a high temperature of 60 °C for 30 days, there was no difference in the crystal form, no difference in the characteristics, a slight increase in the total impurities, and no increase in the water content.

After holding at a high humidity of 92.5% RH for 30 days, there was no difference in the crystal form, no difference in the characteristics, a slight increase in the total impurities, and a slight increase in the water content. After holding in an open environment for 30 days under the high humidity condition, the samples showed a weight gain of 0.56% due to moisture absorption, indicating not obvious hygroscopicity.

After holding at a high temperature of 40 °C and a high humidity of 75% RH for 30 days, there was no difference in the crystal form, no difference in the characteristics, a slight increase in the total impurities, and a slight increase in the water content.

Compared with the compound of Formula (I) prepared in Example 1, the crystal form I of the compound of Formula (I) was relatively stable under conditions of high temperature, high humidity, and a combination of high temperature and high humidity.

After holding the crystal form IV of the compound of Formula I at a high temperature of 60 °C for 30 days, there was no difference in the crystal form, no difference in the characteristics, no increase in the total impurities, and no increase in the water content.

After holding at a high humidity of 92.5% RH for 30 days, there was no difference in the crystal form, no difference in the characteristics, a slight increase in the total impurities, and an increase in the water content. After holding in an open environment for 30 days under the high humidity condition, the samples showed a weight gain of 2.0% due to moisture absorption, indicating obvious hygroscopicity.

After holding at a high temperature of 40 °C and a high humidity of 75% RH for 30 days, there was no difference in the crystal form, no difference in the characteristics, no increase in the total impurities, and a slight increase in the water content.

Compared with the compound of Formula (I) prepared in Example 1, the crystal form IV of the compound of Formula (I) was relatively stable under high temperature conditions.

After holding the crystal form V of the compound of Formula I at a high temperature of 60 °C for 30 days, there was no difference in the crystal form, no difference in the characteristics, a slight increase in the total impurities, and no increase in the water content.

After holding at a high humidity of 92.5% RH for 30 days, there was no difference in the crystal form, no difference in the characteristics, a slight increase in the total impurities, and an increase in the water content. After holding in an open environment for 30 days under the high humidity condition, the samples showed a weight gain of 1.1% due to moisture absorption, indicating not obvious hygroscopicity.

After holding at a high temperature of 40 °C and a high humidity of 75% RH for 30 days, there was no difference in the crystal form, no difference in the characteristics, a slight increase in the total impurities, and a slight increase in the water content.

Compared with the compound of Formula (I) prepared in Example 1, the crystal form V of the compound of Formula (I) was relatively stable under high temperature conditions.

In conclusion, when holding in an open environment, the crystal form I of the compound of Formula (I) is relatively stable under conditions of high temperature, high humidity, and a combination of high temperature and high humidity, and the crystal form IV and the crystal form V are relatively stable under high temperature conditions.

Additionally, in the experiments and further researches, the inventor also discovered:
The crystal form II can be obtained by first cooling in a solvent system of cyclohexane and toluene followed by volatilization. After drying the crystal form II obtained from the volatilization at room temperature under vacuum, the crystal form of the sample was still the crystal form II, indicating a high stability of the crystal form II.

The crystal form III can be obtained by stirring, precipitation and crystallization at 10 °C in a system of methanol/isopropyl ether for a long time, and it is understood by those skilled in the art that the crystal form III has a high stability.

The crystal form VIII is an unstable crystal form, which would undergo thermotropic transition during the post-treatment process (drying at room temperature under vacuum) to turn into the crystal form V. It can be known that the crystal form V is finally obtained from the transition of an intermediate metastable crystal form (the crystal form VIII) and thus has higher stability.

### Example 16: Thermotropic transition experiment

With different crystal forms as starting materials, the samples were placed on a BTS500 heating stage and subjected to XRPD test at room temperature, followed by heating to a setting temperature at a rate of 10 °C/min. After keeping at this temperature for 10 min, XRPD test was conducted again, and then the samples were cooled back to room temperature and subjected to another XRPD test. The results were as shown in Table 4.

**Table 4. Results of thermotropic transition**

| Crystal forms of the sample | Target temperature (°C) | Result |
|---|---|---|
| Crystal form V | 110 | Crystal form V |
| Crystal form VI | 100 | Crystal form VI |
| Crystal form II | 105, 120, 160 | 105°C: crystal form I |
| | | 120°C: crystal form I |
| | | 160°C: crystal form I |

Based on the above experimental results, it can be seen that the crystal forms V and VI have higher stability under the heating condition, and the crystal form II will be transformed into the crystal form I under the heating condition, indicating that the crystal form I has higher stability under the high temperature condition.

It is further demonstrated that the crystal form II can also be used as an intermediate crystal form to further prepare some of the other stable crystal forms as described in the present application. Therefore, the crystal forms of the compound of Formula (I) in the present application has at least one of the effects, such as stability, thus providing multiple intermediate and/or active pharmaceutical ingredient choices for the scale production of active pharmaceutical ingredients and the downstream process (e.g., formulation process) of pharmaceutical products.

The above embodiments are preferred embodiments of the present application, but the embodiments of the present application are not limited by the above embodiments, and any other changes, modifications, substitutions, combinations, and simplifications made without departing from the essence and principle of the present application shall be equivalent replacements, and are included in the scope of protection of the present application.

## Claims

1. A crystal form I of a compound of Formula (I), 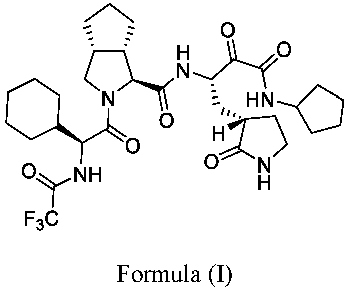 wherein, there are diffraction peaks at 2θ of 10.0, 10.6, 11.5, 12.1, 14.1, 16.7, 17.4, 19.0, 19.4, 20.5, 21.9, 24.9 (±0.2°) in an X-ray powder diffraction (XRPD) pattern of the crystal form I of the compound of Formula (I); further, there are additional diffraction peaks at 2θ of 13.4, 14.5, 17.8, 18.7, 20.0, 21.1, 22.8, 23.8, 26.0, 26.9 (±0.2°) in the XRPD pattern of the crystal form I of the compound of Formula (I); further, the diffraction peak profile in the XRPD pattern of the crystal form I of the compound of Formula (I) is as shown in the table below:
| No. | 2θ (±0.2°) | No. | 2θ (±0.2°) |
|---|---|---|---|
| 1 | 10.0 | 16 | 21.1 |
| 2 | 10.6 | 17 | 21.9 |
| 3 | 11.5 | 18 | 22.8 |
| 4 | 12.1 | 19 | 23.8 |
| 5 | 13.4 | 20 | 24.3 |
| 6 | 14.1 | 21 | 24.9 |
| 7 | 14.5 | 22 | 25.4 |
| 8 | 16.7 | 23 | 26.0 |
| 9 | 17.4 | 24 | 26.9 |
| 10 | 17.8 | 25 | 27.3 |
| 11 | 18.7 | 26 | 28.3 |
| 12 | 19.0 | 27 | 28.7 |
| 13 | 19.4 | 28 | 29.2 |
| 14 | 20.0 | 29 | 29.9 |
| 15 | 20.5 | | |
and further, the XRPD pattern of the crystal form I of the compound of Formula (I) is essentially as shown in Figure 1.

2. The crystal form I of the compound of Formula (I) according to claim 1, wherein, there is a starting point of an endothermic peak at 188.1±3 °C in a differential scanning calorimetry (DSC) pattern of the crystal form I of the compound of Formula (I); and further, the DSC pattern of the crystal form I of the compound of Formula (I) is as shown in Figure 2.

3. The crystal form I of the compound of Formula (I) according to claim 1, wherein, there is a weight loss of 0.3±1% at 100 °C in a thermal gravimetric analysis (TGA) pattern of the crystal form I of the compound of Formula (I); and further, the TGA pattern of the crystal form I of the compound of Formula (I) is as shown in Figure 3.

4. A crystal form II of a compound of Formula (I), 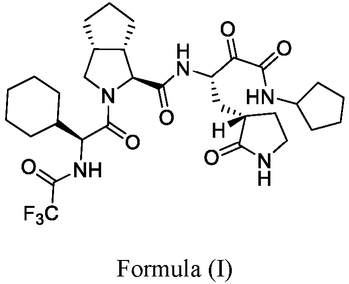 wherein, there are diffraction peaks at 2θ of 10.9, 12.1, 16.1, 17.0, 17.5, 18.3, 23.4 (±0.2°) in an X-ray powder diffraction (XRPD) pattern of the crystal form II of the compound of Formula (I); further, the diffraction peak profile in the XRPD pattern of the crystal form II of the compound of Formula (I) is as shown in the table below:
| No. | 2θ (±0.2°) | No. | 2θ (±0.2°) |
|---|---|---|---|
| 1 | 10.9 | 5 | 17.5 |
| 2 | 12.1 | 6 | 18.3 |
| 3 | 16.1 | 7 | 23.4 |
| 4 | 17.0 | | |
and further, the XRPD pattern of the crystal form II of the compound of Formula (I) is essentially as shown in Figure 5.

5. The crystal form II of the compound of Formula (I) according to claim 4, wherein, there are starting points of endothermic peaks at 72.7, 115.0, 177.3, 262.0 (±3 °C) and a starting point of an exothermic peak at 150.8±3 °C in a differential scanning calorimetry (DSC) pattern of the crystal form II of the compound of Formula (I); and further, the DSC pattern of the crystal form II of the compound of Formula (I) is as shown in Figure 6.

6. The crystal form II of the compound of Formula (I) according to claim 4, wherein, there is a weight loss of 7.8±1% at 230 °C in a thermal gravimetric analysis (TGA) pattern of the crystal form II of the compound of Formula (I); and further, the TGA pattern of the crystal form II of the compound of Formula (I) is as shown in Figure 7.

7. A crystal form III of a compound of Formula (I), 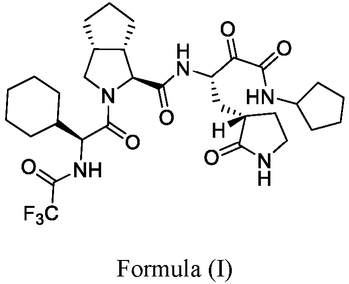 wherein, there are diffraction peaks at 2θ of 6.1, 10.6, 12.3, 16.6, 17.9, 18.7 (±0.2°) in an X-ray powder diffraction (XRPD) pattern of the crystal form III of the compound of Formula (I); further, the diffraction peak profile in the XRPD pattern of the crystal form III of the compound of Formula (I) is as shown in the table below:
| No. | 2θ (±0.2°) | No. | 2θ (±0.2°) |
|---|---|---|---|
| 1 | 6.1 | 4 | 16.6 |
| 2 | 10.6 | 5 | 17.9 |
| 3 | 12.3 | 6 | 18.7 |
and further, the XRPD pattern of the crystal form III of the compound of Formula (I) is essentially as shown in Figure 8.

8. The crystal form III of the compound of Formula (I) according to claim 7, wherein, there are starting points of endothermic peaks at 86.0, 130.8, 186.3 (±3 °C) in a differential scanning calorimetry (DSC) pattern of the crystal form III of the compound of Formula (I); and further, the DSC pattern of the crystal form III of the compound of Formula (I) is as shown in Figure 9.

9. The crystal form III of the compound of Formula (I) according to claim 7, wherein, there is a weight loss of 6.3±1% at 100 °C and a weight loss of 4.5±1% at 200 °C in a thermal gravimetric analysis (TGA) pattern of the crystal form III of the compound of Formula (I); and further, the TGA pattern of the crystal form III of the compound of Formula (I) is as shown in Figure 10.

10. A crystal form IV of a compound of Formula (I), 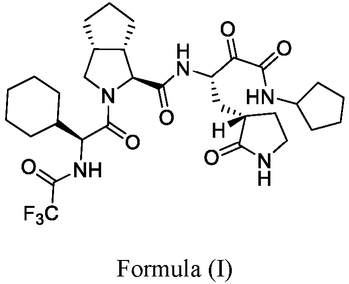 wherein, there are diffraction peaks at 2θ of 6.2, 8.1, 10.7, 16.4, 17.1, 18.6, 19.4, 21.3 (±0.2°) in an X-ray powder diffraction (XRPD) pattern of the crystal form IV of the compound of Formula (I); further, there are additional diffraction peaks at 2θ of 7.1, 11.2, 16.7, 17.7, 18.8, 19.8, 20.4, 22.0, 22.3, 22.5, 23.1, 24.7 (±0.2°) in the XRPD pattern of the crystal form IV of the compound of Formula (I); further, the diffraction peak profile in the XRPD pattern of the crystal form IV of the compound of Formula (I) is as shown in the table below:
| No. | 2θ (±0.2°) | No. | 2θ (±0.2°) |
|---|---|---|---|
| 1 | 6.2 | 14 | 18.6 |
| 2 | 7.1 | 15 | 18.8 |
| 3 | 8.1 | 16 | 19.4 |
| 4 | 10.7 | 17 | 19.8 |
| 5 | 11.2 | 18 | 20.4 |
| 6 | 11.9 | 19 | 21.3 |
| 7 | 12.8 | 20 | 22.0 |
| 8 | 13.4 | 21 | 22.3 |
| 9 | 14.1 | 22 | 22.5 |
| 10 | 16.4 | 23 | 23.1 |
| 11 | 16.7 | 24 | 24.7 |
| 12 | 17.1 | 25 | 27.0 |
| 13 | 17.7 | 26 | 28.4 |
and further, the XRPD pattern of the crystal form IV of the compound of Formula (I) is essentially as shown in Figure 12.

11. The crystal form IV of the compound of Formula (I) according to claim 10, wherein, there are starting points of endothermic peaks at 49.6, 130.5 (±3 °C) in a differential scanning calorimetry (DSC) pattern of the crystal form IV of the compound of Formula (I); and further, the DSC pattern of the crystal form IV of the compound of Formula (I) is as shown in Figure 13.

12. The crystal form IV of the compound of Formula (I) according to claim 10, wherein, there is a weight loss of 3.8±1% at 110 °C and a weight loss of 4.2±1% at 190 °C in a thermal gravimetric analysis (TGA) pattern of the crystal form IV of the compound of Formula (I); further, the TGA pattern of the crystal form IV of the compound of Formula (I) is as shown in Figure 14.

13. A crystal form V of a compound of Formula (I), 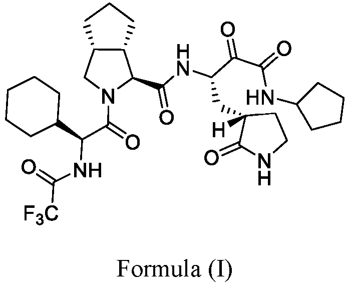 Wherein, there are diffraction peaks at 2θ of 6.2, 8.1, 10.6, 16.2, 18.5, 19.1 (±0.2°) in an X-ray powder diffraction (XRPD) pattern of the crystal form V of the compound of Formula (I); further, the diffraction peak profile in the XRPD pattern of the crystal form V of the compound of Formula (I) is as shown in the table below:
| No. | 2θ (±0.2°) | No. | 2θ (±0.2°) |
|---|---|---|---|
| 1 | 6.2 | 4 | 16.2 |
| 2 | 8.1 | 5 | 18.5 |
| 3 | 10.6 | 6 | 19.1 |
and further, the XRPD pattern of the crystal form V of the compound of Formula (I) is essentially as shown in Figure 16.

14. The crystal form V of the compound of Formula (I) according to claim 13, wherein, there is a starting point of an endothermic peak at 148.4±3 °C in a differential scanning calorimetry (DSC) pattern of the crystal form V of the compound of Formula (I); and further, the DSC pattern of the crystal form V of the compound of Formula (I) is as shown in Figure 17.

15. The crystal form V of the compound of Formula (I) according to claim 13, wherein, there is a weight loss of 10.5±1% at 190 °C in a thermal gravimetric analysis (TGA) pattern of the crystal form V of the compound of Formula (I); and further, the TGA pattern of the crystal form V of the compound of Formula (I) is as shown in Figure 18.

16. A crystal form VI of a compound of Formula (I), 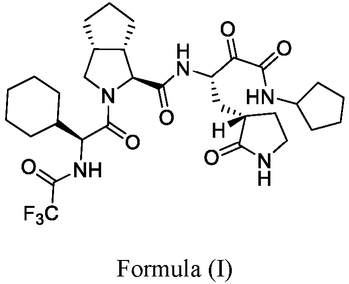 wherein, there are diffraction peaks at 2θ of 5.5, 5.8, 10.7, 16.9, 17.9, 18.3 (±0.2°) in an X-ray powder diffraction (XRPD) pattern of the crystal form VI of the compound of Formula (I); further, there are additional diffraction peaks at 2θ of 9.1, 10.4, 15.9, 20.0, 20.9, 21.6 (±0.2°) in the XRPD pattern of the crystal form VI of the compound of Formula (I); further, the diffraction peak profile in the XRPD pattern of the crystal form VI of the compound of Formula (I) is as shown in the table below:
| No. | 2θ (±0.2°) | No. | 2θ (±0.2°) |
|---|---|---|---|
| 1 | 5.5 | 7 | 16.9 |
| 2 | 5.8 | 8 | 17.9 |
| 3 | 9.1 | 9 | 18.3 |
| 4 | 10.4 | 10 | 20.0 |
| 5 | 10.7 | 11 | 20.9 |
| 6 | 15.9 | 12 | 21.6 |
and further, the XRPD pattern of the crystal form VI of the compound of Formula (I) is essentially as shown in Figure 20.

17. The crystal form VI of the compound of Formula (I) according to claim 16, wherein, there is a starting point of an endothermic peak at 134.2, 184.3 (±3 °C) in a differential scanning calorimetry (DSC) pattern of the crystal form VI of the compound of Formula (I); and further, the DSC pattern of the crystal form VI of the compound of Formula (I) is as shown in Figure 21.

18. The crystal form VI of the compound of Formula (I) according to claim 16, wherein, there is a weight loss of 16.2±1% at 200 °C in a thermal gravimetric analysis (TGA) pattern of the crystal form VI of the compound of Formula (I); and further, the TGA pattern of the crystal form VI of the compound of Formula (I) is as shown in Figure 22.

19. A crystal form VII of a compound of Formula (I), 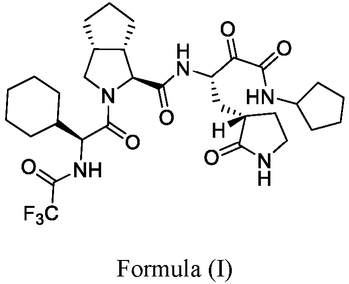 wherein, there are diffraction peaks at 2θ of 6.3, 10.9 (±0.2°) in an X-ray powder diffraction (XRPD) pattern of the crystal form VII of the compound of Formula (I); and further, the XRPD pattern of the crystal form VII of the compound of Formula (I) is essentially as shown in Figure 23.

20. The crystal form VII of the compound of Formula (I) according to claim 19, wherein, there is a starting point of an endothermic peak at 130.2±3 °C in a differential scanning calorimetry (DSC) pattern of the crystal form VII of the compound of Formula (I); and further, the DSC pattern of the crystal form VII of the compound of Formula (I) is as shown in Figure 24.

21. The crystal form VII of the compound of Formula (I) according to claim 19, wherein, there is a weight loss of 8.6±1% at 190 °C in a thermal gravimetric analysis (TGA) pattern of the crystal form VII of the compound of Formula (I); and further, the TGA pattern of the crystal form VII of the compound of Formula (I) is as shown in Figure 25.

22. An active pharmaceutical ingredient, comprising a compound of Formula (I) and/or a hydrate or a solvate thereof, wherein, the active pharmaceutical ingredient comprises at least one of the crystal forms of the compound of Formula (I) as defined according to any one of claims 1-21;

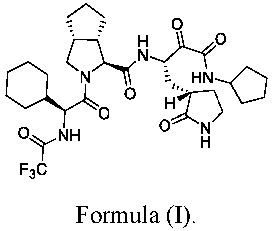

23. A pharmaceutical composition, wherein the pharmaceutical composition comprises a pharmaceutically acceptable excipient and the active pharmaceutical ingredient according to claim 22.

24. The pharmaceutical composition according to claim 23, wherein, the pharmaceutically acceptable excipient comprises at least one of filler, binder, disintegrant, and lubricant.

25. A drug, wherein, the drug comprises at least one of the crystal form of any one of claims 1-21 or the active pharmaceutical ingredient of claim 22 or the pharmaceutical composition of any one of claims 23-24.

26. Use of the crystal form of any one of claims 1-21 or the active pharmaceutical ingredient of claim 22 or the pharmaceutical composition of any one of claims 23-24 in the preparation of a drug for treating coronavirus infection.

27. The method according to claim 26, wherein, the coronavirus is HCoV-229E, HCoV-OC43, HCoV-NL63, HCoV-HKU1, SARS-CoV, MERS-CoV, SARS-CoV-2, or a variant thereof.
